(19) **Europäisches Patentamt / European Patent Office / Office européen des brevets**

(11) **EP 3 346 402 A1**

(12) **EUROPEAN PATENT APPLICATION**

(43) Date of publication:
**11.07.2018 Bulletin 2018/28**

(51) Int Cl.:
**G06F 19/00** *(2018.01)*    *A61B 5/11 (2006.01)*
**G08B 21/04** *(2006.01)*

(21) Application number: **17150270.1**

(22) Date of filing: **04.01.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fraunhofer Portugal Research 4200-135 Porto (PT)**

(72) Inventors:
- **GUIMARÃES, Vânia**
  **P-4200-135 Porto (PT)**
- **SILVA, Joana**
  **P-4200-135 Porto (PT)**

- **SOUSA, Inês**
  **P-4200-135 Porto (PT)**
- **AGUIAR, Bruno**
  **P-4200-135 Porto (PT)**
- **SOUSA, Filipe**
  **P-4200-135 Porto (PT)**
- **ELIAS, Dirk**
  **P-4200-135 Porto (PT)**

(74) Representative: **Zimmermann, Tankred Klaus et al Schoppe, Zimmermann, Stöckeler Zinkler, Schenk & Partner mbB Patentanwälte Radlkoferstrasse 2 81373 München (DE)**

(54) **APPARATUS AND METHOD FOR TRIGGERING A FALL RISK ALERT TO A PERSON**

(57)     An apparatus for triggering a fall risk alert to a person is shown. The apparatus comprises a fall sensor configured to evaluate motions of the person so as to obtain a first fall risk value and a risk calculator configured to determine a second fall risk value based on a set of parameters. Furthermore, an adaption unit may determine weights associated with parameters of the set of parameters, wherein the adaption unit is configured to adapt the weights based on the first fall risk value. The apparatus is further configured to trigger the fall risk alert depending on the second fall risk value.

Fig. 1d

EP 3 346 402 A1

**Description**

Specification

The disclosure relates to an apparatus and a method for triggering a fall risk alert to a person. A point in time to trigger the fall risk alert may be determined using input parameters derived from the person, and/or the environment of the person, and/or input parameters derived from further persons. According to embodiments, the disclosure provides an integrated solution for adaptive multifactorial fall risk assessment, detection and prevention in elderly.

Falls are a major health issue, resulting in severe personal consequences and high healthcare costs. The multifactorial origin of falls is nowadays recognized as well as the reversibility of most fall risk factors by fall prevention strategies that include maintaining an active life style. However, physical activity stimulation and exercise programs should be carefully tailored for individuals in risk of falling, since they are mostly elderly, often frail and may present several co-morbidities.

Mobile solutions for the management of falls in the elderly embrace individual solutions for fall detection, fall risk assessment, fall prevention and activity monitoring that have been technically demonstrated before. However, these methodologies rely on previous knowledge that is confined to what was previously established among several studies with some segments of the population, during limited periods of time. As a result, a person is frequently considered to be at risk of falling when his parameters differ from the normal. In fact, each human being develops his own movement strategies and therefore what is often considered as a risk reveals not to be a cause of a fall. In reality, the impact of individual, personal fall risk factors on the overall fall risk score is time variant, e.g. due to changes in environmental conditions, or the medication taken in a specific time of the day. Current fall related methodologies are essentially static in what concerns to the parameters used, as well as the way they are related. As a result, algorithms and calculations do not adapt to the individual person using the system, leading sometimes to misleading results. Moreover, the interaction between fall-related components is currently not dynamic, which leads to an inability to learn and to adjust the individual parameters to the person. Systems are also unable to deduct new knowledge automatically from the data.

Currently the fall risk assessment and definition of interventions for fall prevention are done by trained professionals in healthcare facilities. However, this approach does not allow continuous or sufficiently frequent monitoring of the evolution of fall risk factors and corresponding adjustment of fall prevention strategies.

Falls risk assessment is a vast research area with widely disparate approaches being used. There are various scoring systems intended for use in hospitals, nursing homes, or outpatient settings. The available scales are designed to be used by different professionals (e.g., geriatric doctors, nurses, or physical therapists), and are based on questionnaires, observations, physical examinations, or their combination.

Possible fall risk assessment tests do not comprise all the factors that are known to be related to the risk of falling, due to time constrains and difficulties in measuring some of these factors in a sporadic assessment.

Fall prevention plans are based on generic recommendations regarding house and environment dangers, certain activities and movements that may cause falls. High risk cases are referred to a few sessions of physiotherapy or fall prevention exercise groups, in these approaches the individuals are accompanied by trained professionals, which implies costs to the user or healthcare system. Moreover, in group sessions the exercises are not specific for each individual.

Several technological solutions have been proposed for fall detection, fall risk assessment, fall prevention or activity tracking. However, the data collected by these modules is not processed at a higher level deriving dynamic adaptive relations between the parameters monitored and the events detected.

Therefore, there is a need for an improved approach to overcome the aforementioned limitations.

It is an object of the present invention to provide an improved concept for determining fall risks of a person.

Embodiments show an apparatus for triggering a fall risk alert to a person. The apparatus comprises a fall sensor configured to evaluate motions (or movements) of the person so as to obtain a first fall risk value (e.g. an unbalance, a fall probability or a binary variable indicating a fall) and a risk calculator configured to determine a second fall risk value based on a set of user-related and external parameters, wherein the sensitivity of the fall sensor is adjusted using the second fall risk value. Furthermore, the apparatus is configured to trigger a fall risk alert depending on the first and the second fall risk value.

Further embodiments show an apparatus for triggering a fall risk alert to a person. The apparatus comprises a fall sensor configured to evaluate motions of the person so as to obtain a first fall risk value and a risk calculator configured to determine a second fall risk value based on a set of parameters. Furthermore, an adaption unit may determine weights associated with a parameter of the set of parameters as to determine the second fall risk value based on the first fall risk value. The apparatus is further configured to trigger the fall risk alert depending on the second fall risk value.

The present disclosure is based on the finding that a sensitivity of a fall sensor (the sensitivity may be a threshold where a fall or a near-fall is detected) is adjustable using parameters (input to the risk calculator) indicating the probability of a fall to improve the triggering of a fall risk alert. Alternatively, for an improved fall risk alert triggering, an adaptive learning of a classifier, such as the risk calculator, to person-specific falls characteristics may be performed. In other words, falls of the person are detected and used, together with the current values of the parameters related to the fall, to adapt the calculation of the fall sensor. The parameters may influence the probability of falls such as weather conditions, accumulation of falls of further persons at certain locations due to environmental conditions, medication intake or the like.

The fall sensor may detect physical indications of a fall or a near-fall based on the analysis of the motion data of the person such as data from a gyroscope or an acceleration sensor or, it may use alternative sources of information to get those indications, e.g. using image or sound analysis. However, the analysis of the motion data of the person should be evaluated or analyzed to determine whether the person has been falling or is about to fall (i.e. it is shortly before a fall). The evaluation of the sensor data is adjustable based on the information derived from the parameters in that the sensitivity that the person has been falling or was about to fall is changed. Due to this adjustment, the probability of a false alarm, e.g. measured in terms of sensitivity or specificity is adaptable. A higher probability of falling, indicated by the parameters, that a person has been falling or is about to fall may result in a higher sensitivity of the fall sensor to indicate an approaching fall or a current or recent fall. Thus, a problem that may be addressed is the lack of a comprehensive and affordable technological solution for management of the falls problem in elderly.

The adaptive learning may be used to adapt the weights associated with the parameters to determine the second fall risk value. Such an adaptive learning may improve the determination of the second fall risk value, i.e. the actual determination of the fall risk of the person e.g. by way of a percent value. However, the knowledge which regarding the influence of the parameters to the determination of the second fall risk value may vary (most likely improved) over time. This may be performed by various factors such as receiving further (external) knowledge about the influence of a certain medication, by evaluation the falls characteristics of the person or by evaluating and sharing other peoples' falls characteristics (crowdsourcing).

Both improvements may be beneficial especially when referring to issuing alerts and recommendations regarding actions that may prevent future falls early in time (i.e. well before a fall is about to happen). Thus, both improvements provide alternative solutions to the problem referenced above. Furthermore, the improvements relate to a single-concept linked group of inventions in that they both relate to the actual fall detection before determining whether or not to trigger the fall risk alert.

This adjustment of the sensitivity to adapt the false alarm probability is advantageous for the prediction of future falls in that the person is not annoyed if the overall probability of a fall is low but, on the other hand, to not miss an approaching fall e.g. by triggering the fall alert too late (if the person already fell). Therefore, according to embodiments, the apparatus is configured to trigger the fall risk alert if the first fall risk value indicates that the person is about to fall. The sensitivity of the fall sensor may be adjusted based on the second fall risk value to change a set of thresholds of the first fall risk value, wherein the set of thresholds (or more precisely a value of the first risk value exceeding the thresholds) indicates that the person is about to fall.

On the other hand, if the person has been falling, the measured parameters (i.e. measurements of the fall sensor and/or the parameters) may be used to train a classifier to obtain the fall risk, or more general, used for the determination of the fall risk probability. Thus, by adapting the sensitivity, less false alarms are used for determination of the fall risk probability, e.g. the update of the training of the classifier. In other words, a measure of the fall risk probability (this measure may be the subsequently described fall risk score, also referred to as second fall risk value) is used to adapt the physical fall detection (fall sensor) of the person. Thus, according to embodiments, the risk calculator may further adjust the sensitivity of the fall sensor such that an increasing fall probability indicated by the first and the second fall risk value results in a reduced probability that the fall sensor misses to indicate a fall of the person. A processor may be configured to train the risk calculator to falls of the person if the first risk value indicates a fall of the person using the first fall risk value and related parameters of the set of parameters.

Embodiments show that the risk calculator is configured to continuously adjust the sensitivity of the fall sensor to apply a time-variant threshold to the first fall risk value indicating if the person is about to fall or indicating whether the person has been falling. This enables the apparatus to directly adapt the fall sensor to changes of the second fall risk value without a delay. Thus, since physical activity stimulation and exercise programs should be carefully tailored to prevent the person from falling, it is advantageous to continuously monitor the elderlies at risk of falling, analyzing their normal daily activity, movements, medication, falls as well as environment and context data, in order to compute a dynamic fall risk status and use all the information retrieved to suggest fall prevention strategies.

Therefore, according to further embodiments, the apparatus is configured to provide the person with fall prevention recommendations based on the first or the second fall risk value. The fall prevention recommendations may comprise one or a combination of the following recommendations: performing a medical check-up, performing a diet, following a provided nutrition plan, performing sports activities, modifying the home environment. Thus, using this user (or person) specific fall prevention recommendations, the person is guided to perform the respective activities according to their physical strength. Therefore, embodiments show the apparatus configured to monitor the person during performing the activities and to monitor the evolution of the activities to guide the person accordingly.

Further embodiments show the apparatus using information from further persons in order to improve the determination of the first and/or the second fall risk value. This enlarges the dataset that can be used for training to e.g. improve the adaption of the risk calculator to patterns within values of parameters of the set of parameters in close relation to a fall, since a fall should be a seldom event in a person's life. Additionally, the fall sensor (i.e. a model to determine the first fall risk value) may be further adapted using the enlarged dataset including further persons. In other words, the apparatus is configured to obtain first fall risk values and sets of parameters from further persons different from the person and to adapt a determination of the first or the second fall risk value based on the first fall risk values from the further persons and respective parameters of the sets of parameters from the further persons.

The configuration of the described apparatuses may perform a corresponding method optionally being implemented in a computer program.

Before embodiments are described in detail using the accompanying figures, it is to be pointed out that the same or functionally equal elements are given the same reference numbers in the figures and that a repeated description for elements provided with the same reference numbers is omitted. Hence, descriptions provided for elements having the same reference numbers are mutually exchangeable.

Embodiments of the present invention will be discussed subsequently referring to the enclosed drawings, wherein:

Fig. 1 a shows a schematic block diagram of an apparatus for triggering a fall risk alert to a person according to embodiments;

Fig. 1b shows a schematic block diagram of the apparatus for triggering a fall risk alert to a person according to embodiments;

Fig. 1c shows a schematic block diagram of the apparatus for triggering a fall risk alert to a person according to embodiments;

Fig. 1d shows a schematic block diagram of the apparatus for triggering a fall risk alert to a person according to embodiments using adaptive learning;

Fig. 2a shows a schematic block diagram of the apparatus according to embodiments using adaptive learning and a sensitivity adjustment of the fall sensor;

Fig. 2b shows a schematic block diagram of the apparatus for triggering a fall risk alert to a person according to embodiments in more detail;

Fig. 3 shows a schematic block diagram of a data server according to embodiments;

Fig. 4 shows a schematic block diagram of a device for measuring (values of) parameters of the set of parameters and/or the motion of the person, the device may include sensing, processing, communication and storage capabilities and may further comprise a user interface to interact with the user.

Fig. 5 shows exemplary diagrams of the fall risk factor over different (values of) a parameter of the set of parameters;

Fig. 6 shows a schematic block diagram of a general scheme for a weights update mechanism using an adaptive learning mechanism;

Fig. 7 shows a schematic diagram of an adaptive weighting update mechanism according to a first example;

Fig. 8 shows a schematic diagram of an adaptive weighting update mechanism according to a second example;

Fig. 9 shows a schematic diagram of parameters and a respective fall risks monitoring;

Fig. 10 shows a schematic block diagram of a general scheme of a fall risk-related adaptive learning mechanism;

Fig. 11 shows a schematic diagrams of the adaption of weights after an update of the weights;

Fig. 12 shows a schematic diagram of a fall sensor sensitivity adjustment;

Fig. 13 shows a schematic diagram of a fall prevention mechanism;

Fig. 14 shows a schematic diagram of a fall prevention mechanism for long-term interventions;

Fig. 15 shows a schematic diagram of multiple apparatuses using crowdsourcing locations of frequent falls, associated with a time of occurrence and context to deduct environmental risk factors; and

Fig. 16 shows a schematic diagram of mapping parameters to risks using crowdsource-based mapping rules update.

In the following, embodiments of the invention will be described in further detail. Elements shown in the respective figures having the same or a similar functionality will have associated therewith the same reference signs.

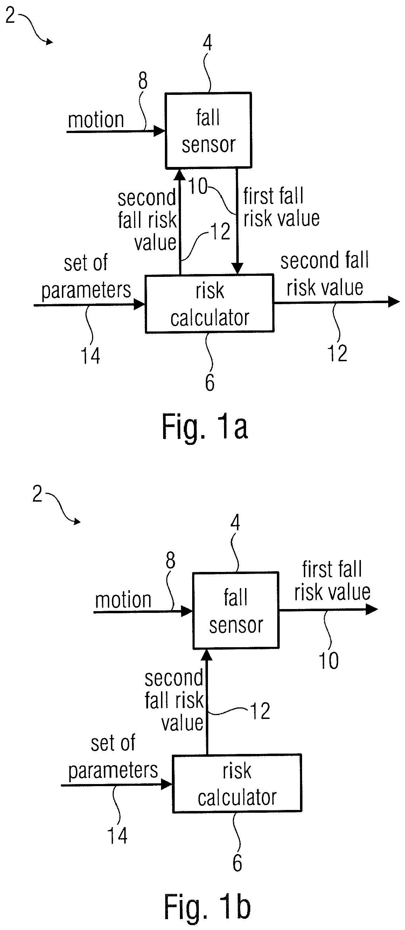

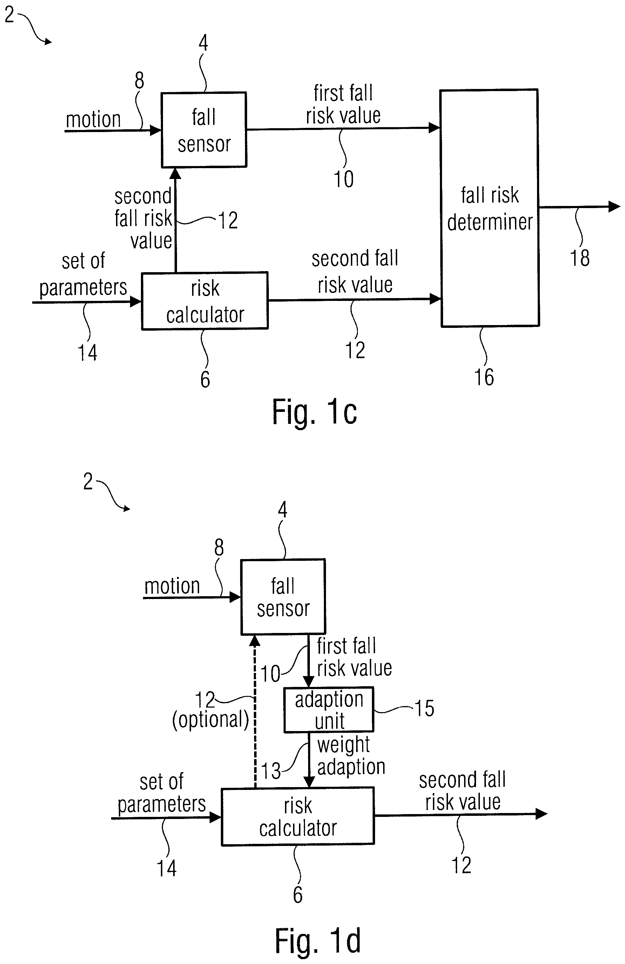

Fig. 1a, 1b and 1c show an apparatus 2 for triggering a fall risk alert to a person using sensitivity adjustment of the fall sensor wherein Fig. 1d illustrates adaptive learning of the classifier. The apparatus comprises a fall sensor 4 and a risk calculator 6. The fall sensor 4 may evaluate motions 8 of the person so as to obtain a first fall risk value 10. The risk calculator 6 may determine a second fall risk value 12 based on a set of parameters 14, wherein a sensitivity of the fall sensor 4 is adjusted using the second fall risk value 12. Thus, the fall sensor 4 receives input from the risk calculator 6 in terms of the second fall value 12. The fall sensor may determine motions of the person by e.g. evaluating sensor data obtaining physical characteristics of the person such as gait speed, step length, unusual/higher accelerations etc., to obtain whether the person is about to fall or whether the person has been falling. The set of parameters 14, input to the risk calculator 6, may comprise different parameters such as environmental conditions (e.g. temperature, humidity etc.), personal data (e.g. age, activities, medication etc.), relating indirectly or in a medium term to a fall wherein the sensor data obtained from the fall sensor may be directly related to falls (since falls are measurable by the fall sensor, the risk calculator may not be able to measure that a person has been falling). However, the motions 8 input to the fall sensor may be also parameters of the set of parameters input to the risk calculator. Furthermore, the apparatus is configured to trigger a fall risk alert depending on the first and the second fall risk value.

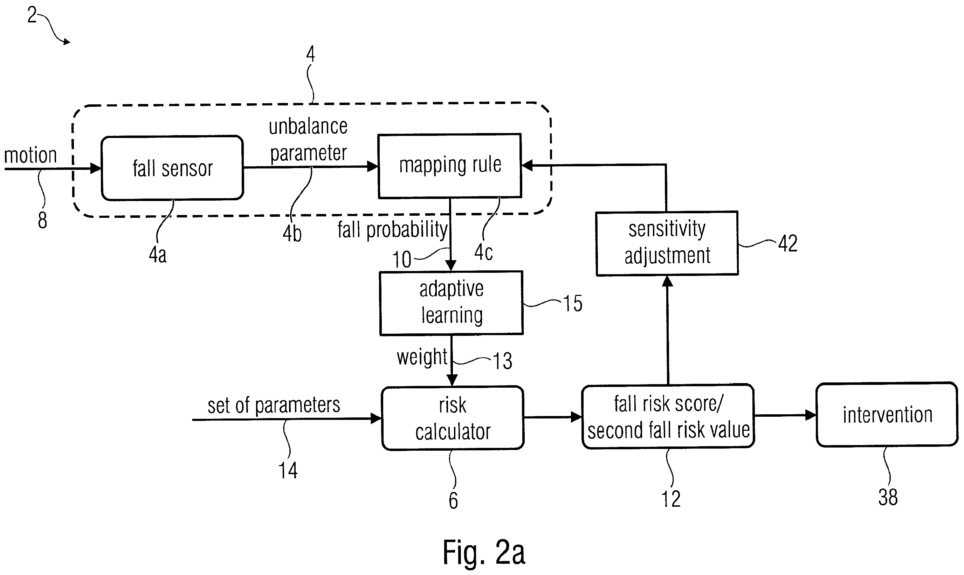

Optionally, to derive at an embodiment using the sensitivity adjustment and adaptive learning, the apparatus comprises a processor configured to adapt a weight associated with a parameter of the set of parameters to determine the second fall risk value based on the first fall risk value. The fall risk alert may be triggered depending on the first and the second fall risk value by triggering the fall risk alert based on the second fall risk value adapted by the first fall risk value. Further details may be shown with respect to Fig. 2a.

Nonetheless, Fig. 1a, 1b and 1c differ in that they show different embodiments of how the apparatus triggers the fall risk alert depending on the first and the second fall risk value. Fig. 1 a shows the apparatus 2 according to embodiments where the second fall risk value indicates whether or not to trigger the fall risk alert. However, the determination of the second fall risk value is dependent on the first fall risk value 10 since the same is input to the risk calculator 6. As one embodiment, the determination of the second fall risk value from the set of parameters is adjusted using e.g. training or updating the risk calculator 6, e.g. a classifier, with a subset of recent first fall risk values that indicate that the person has been falling and corresponding (values of the) parameters of the set of parameters present at the same time. Hence, the second fall risk value is determined based on the first fall risk value and therefore, the apparatus is configured to trigger the fall risk alert depending on the first and the second fall risk value, even though the actual output to decide whether or not to trigger the fall risk alert is the second fall risk value. It should be noted that the term parameter relates to the variable as well as to an actual value of the variable.

Fig. 1b shows a further embodiment where the first fall risk value initiates the triggering of the fall risk alert. Therefore, the fall sensor determines whether the person is about to fall, in contrast to the embodiments described with respect to Fig. 1b, where the fall sensor determines an actual fall of the person for training the risk calculator to parameter value patterns of the set of parameters related to falls (i.e. patterns before and/or during a fall). By determining whether the person is about to fall, the triggering of the fall risk alert may be performed by the fall sensor in advance of a fall. However, by adjusting the sensitivity of the fall sensor using the second fall risk value, the number of false alarms may be reduced if the second fall risk value indicates a low probability that a fall of the person occurs.

In this case, a threshold that may be applied to the first fall risk value in order to decide whether or not a fall is about to occur depends on (or is adjusted by) the second fall risk value and therefore a probability that a person may fall. A higher probability that a person is about to fall, indicated by the second fall risk value, may therefore result in an increased sensitivity of the fall sensor to detect patterns in the data of the sensors providing the information regarding the motions 8 that refer to an approaching fall. In this case, a higher false alarm probability may be approved, since the overall chance of falling is also increased. Thus, the apparatus 2 may trigger the fall risk alert based on the first fall risk value, wherein a threshold at the first threshold value indicating whether or not a person is about to fall is adjusted using the second fall risk value. Therefore, the apparatus is configured to trigger the fall risk alert depending on the first and the second fall risk value, even though the actual output to decide whether or not to trigger the fall risk alert is the first fall risk value.

Fig. 1c provides extended embodiments of the embodiments of Fig. 1 b. Additionally to the embodiments described with respect to Fig. 1b, the apparatus is configured to trigger the fall risk alert depending on the first and the second fall risk value in that the first and the second fall risk value provide a weighted input to the decision whether or not the person is about to fall. The decision, or the determination, whether or not the person is about to fall may be performed by a fall risk determiner 16 such as a processor. The fall risk determiner may therefore receive the first fall risk value 10 and the second fall risk value 12. Furthermore, the fall risk determiner may perform a weighted combination of the first and the second fall risk value to obtain a measure 18 whether or not to trigger the fall risk alert to the person.

Fig. 1d shows an apparatus 2 for triggering a fall risk alert to a person. The apparatus comprises the fall sensor 4, the risk calculator 6 and a adaption unit 15.

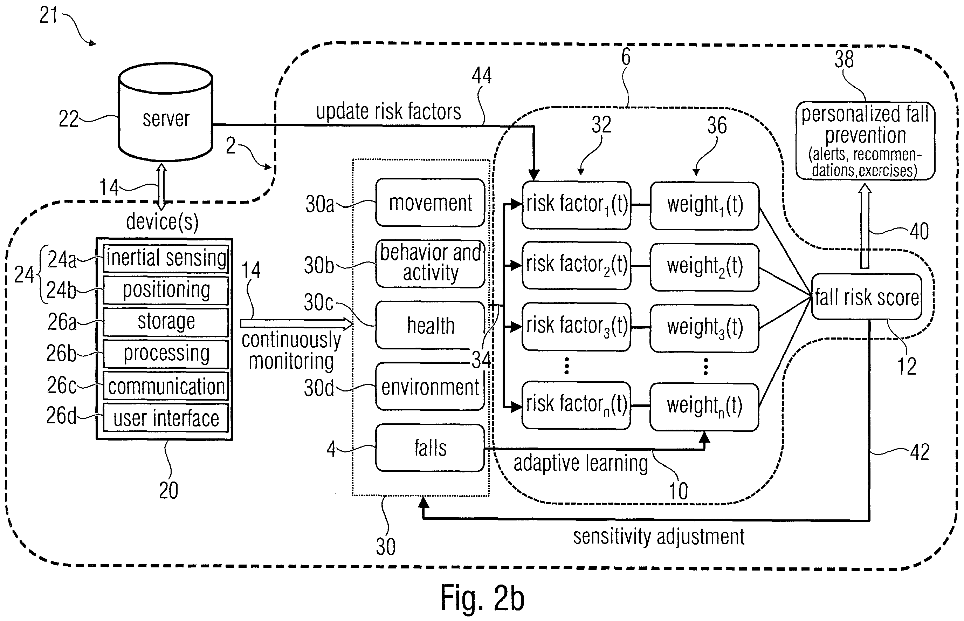

The fall sensor may evaluate motions of the person so as to obtain the first fall risk value 10. The risk calculator 6 may determine a second fall risk value based on the set of parameters 14. The fall sensor and the risk calculator may be connected by the adaption unit 15 configured to adapt a weight associated with a parameter of the set of parameters 14b as to determine the second fall risk value based on the first fall risk value. Therefore, the adaption unit may use the first fall risk value 10, e.g. when the same indicates a fall of the person, to determine a weight adaption value 13. The adaption unit 15 may be implemented in a processor. A parameter may refer to balance, strength, medication, weather, etc. Using mapping rules, the parameter or the set of parameters may be converted to one fall risk factor or a set of fall risk factors. The weight associated with the parameter of the set of parameters may be a weight attributed to a fall risk factor. A fall risk factor may comprise one parameter or a weighted combination of parameters to obtain an indicator for a risk of a person to fall (c.f. Fig. 2b, 32, 36). The actual second fall risk value may be a weighted (linear or higher order) combination of two or more fall risk factors, wherein these weights (36 in Fig. 2b) applied to the fall risk factors may be adapted by the adaption unit 15 value.

Optionally, the adaption unit may receive the set of parameters 14 (or values thereof) related to a fall of the person by performing the adaptive learning in the adaption unit 15 based on the respective parameters related to the fall and optionally to balance the training set, using further parameters not related to a fall. Finally, the apparatus is configured to trigger the fall risk alert depending on the second fall risk value (adapted by the first fall risk value).

Optionally, to derive at an embodiment using the sensitivity adjustment and adaptive learning, the sensitivity of the fall sensor is adjusted using the second fall risk value.

So far, the described embodiments are described in parallel. In the following, however, the figures are described according to the embodiments of Fig. 1a and 1d. Nonetheless, they may be adapted to the embodiments of Fig. 1b or Fig. 1c according to the description above.

According to embodiments, the apparatus 2, using e.g. the risk calculator 6, may trigger the fall risk alert comprising recommendations for the user to prevent future falls dependent on the second fall risk value and optionally independent from a current value of the first fall risk value. Even though the second fall risk value, or more specifically the determination of the second fall risk value, may be influenced by the first fall risk value e.g. through the adaptive learning applied by the adaption unit, the fall risk calculator does not explicitly use the first fall risk value to trigger the fall risk alert. Thus, the fall risk alert may be triggered independently from the first fall risk value.

Fig. 2a shows a schematic block diagram according to embodiments. Herein, the sensitivity adjustment using feedback loop 42 and the adaptive learning using adaption unit 15 may both be performed. Additionally, an embodiment of the fall sensor is shown, which is generally applicable, not only to the embodiment of the apparatus comprising both, adaptive learning and sensitivity adjustment. According to the embodiment, the fall sensor 4 comprises one or more fall sensing units 4a such as a gyroscope or an acceleration sensor providing unbalance parameters 4b related e.g. to acceleration peaks, rapid changes in orientation or an unbalance of the person. These unbalance parameters may be applied to a mapping rule 4c to determine the (i.e. one) first fall risk value 10 based on the multiple unbalance parameters according to the mapping rule. Output of the mapping rule is the first fall risk value which may be a binary variable indicating whether or not the person fell or a relative variable indicating the likelihood or probability of the person to fall e.g. in the range of 0% to 100%.

Thus, when applying the sensitivity adjustment to the fall sensor via feedback loop 42, the sensitivity adjustment may be applied to the mapping rule 4c such that the mapping rules are adapted according to the current second fall risk value 12 (i.e. the fall risk score). In other words, the risk calculator may continuously adjust the sensitivity of the fall sensor 4 to apply a time-variant threshold to the first fall risk value indicating if the person has been falling.

The risk calculator, when determining the second fall risk value 12, may determine a trend within consecutive values of parameters of the set of parameters and to trigger the fall risk alert comprising fall prevention recommendations if the trend within the consecutive values of the parameters indicates an increasing fall risk probability. This trend (or drift), which may be referred to as a slowly varying, basically increasing or decreasing value of one or more parameters, may indicate a constitutional change of the person, a change of the environment etc. If change refers to the persons constitution, the trend may be in the order or months or even years wherein, if e.g. the weather conditions change, the trend may be comparably quick if it is e.g. referred to a start of rain and an increasing slippery of a certain (wet) floor. Each of these trends may result in a different recommendation to prevent the person from falling, i.e. e.g. exercises to increase the persons' strength to stop the constitutional change (or decrease) of the person or in avoiding places being slippery if the floor is wet.

Additionally or alternatively, the risk calculator may trigger the fall risk alert if one parameter or combination of parameters of the set of parameters (or, more precisely, one fall risk factor or a set of fall risk factors calculated from one or a set of parameters using the mapping rules) exceeds a threshold (value), wherein the threshold indicates an increasing fall risk probability. Thus, e.g. a recommendation for fall prevention being a signal that is optionally (part of) the fall risk alert, may be triggered by a single fall risk factor exceeding the threshold. Therefore, the combination, also referred to as fall risk factor, may be a weighted combination (also referred to as mapping rules) such as e.g. a linear combination of two or more parameters of the set of parameters. However, one fall risk factor may also comprise only one parameter instead of a weighted combination of parameters. A mapping rule may be referred to as how one or a set of parameters can be translated into a fall risk factor. Please note that mapping rules may be provided in the fall sensor (cf. 4c) and/or to define the risk factors (cf. Fig. 2b). For practical reasons, the term "parameter" may be used interchangeably along the text to refer to a fall risk factor, when the need of distinguishing both concepts is not necessary to understand the underlying ideas of the text.

Fig. 2b shows a schematic block diagram of a system 21 comprising a data server 22 and the apparatus 2 comprising one or more devices 20. The device 20 may comprise hardware components such as one or more sensors 24, e.g. an inertial sensor (e.g. gyroscope) or a position sensor (e.g. GPS: global positioning system). Further sensors may be a barometer, a temperature sensor, acceleration sensor, pulse monitor, blood pressure monitor, biometric sensor, galvanic sensor, pulse oximeter etc. In other words, the fall sensor may evaluate motions of the person using values/parameters of at least one or a combination of the following sensors: gyroscope, acceleration sensor, pulse monitor, blood pressure monitor, biometric sensor, galvanic sensor, pulse oximeter.

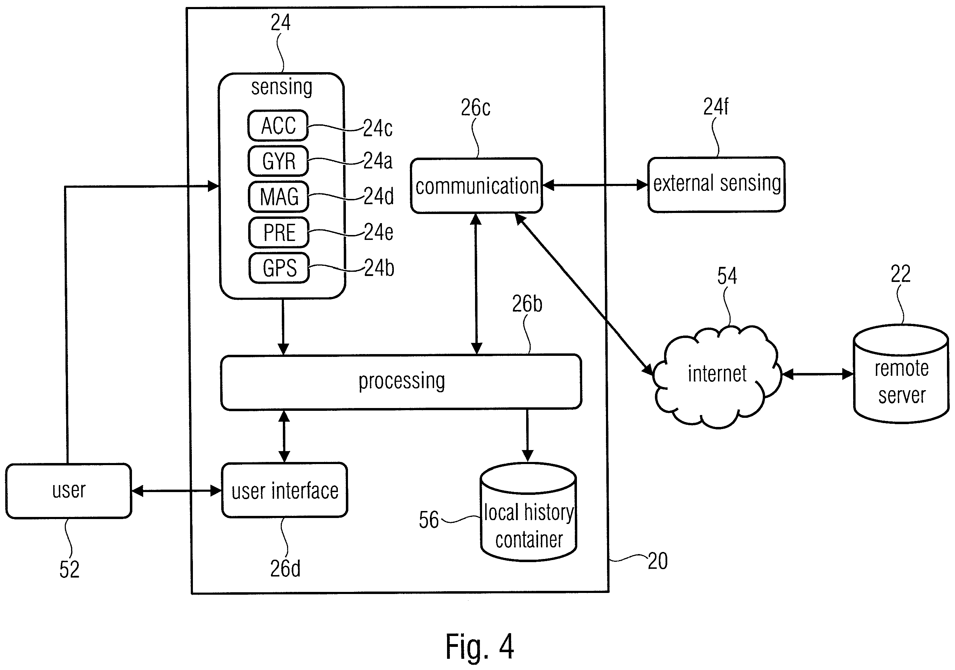

Furthermore, the device may comprise a storage 26a, a processor 26b, a communication entity such as a network (e.g. a WLAN: wireless local area network) module 26c and/or a user interface 26d. For further explanation of the device, it is referred to the description of Fig. 4.

Basically, the device may be configured to gather all relevant information from the person. This information, i.e. the set of parameters 14, may be continuously monitored and provided to the processing that may be performed in processor 26b or in a further processor of the apparatus or alternatively at least partially implemented in hardware. Fig. 2b provides a categorization of the information (or data, values of the parameters) in block 30. Block 30 may comprise parameters related to movement 30a, behavior and activity 30b, health 30c, environment 30d and the fall sensor 4. However, this categorization is optional since the parameters to be categorized are already provided by the device 20. However, the fall sensor 4 is advantageously implemented in order to obtain actual falls of the person independent from other parameters. Thus, according to embodiments, the blocks 30a to 30d provide a (optionally non-physical and therefore for illustrative purposes in this disclosure only) grouping of the parameters. Additionally or alternatively, one or more blocks 30a to 30d may perform a pre-processing of the parameters corresponding to the respective block, e.g. to obtain one output indicating the health status when exemplary referring to block 30c.

A further stage comprises multiple (fall) risk factors 32. A risk factor may be one parameter or a weighted combination of parameters that provides insight into the probability of falling, indicated by arrows 34. These arrows may represent the mapping rules, such as a (linear or higher degree) combination of one or more parameters. For example, the intake of a certain medicine in combination with an increased pulse rate may not relate to an increased probability of falling since this is a side effect of the certain medicine wherein an increased pulse rate without the intake of the certain medicine may have other reasons that may relate to a higher probability of falling. Thus, one risk factor may comprise the parameters (or during measurement the respective parameters) whether the certain medicine was taken and the pulse rate, either as a binary value whether or not the pulse rate exceeded a certain threshold or as a continuous value. Any other combination of parameters is also possible. Thus, the risk factors 32 may be an input stage of a classifier.

A subsequent stage comprises one or more weights 36. The number of weights may be equal to, less than, or greater than the number of risk factors 32. The weights 36 provide the weighting of the influence of a certain risk factor or a combination of risk factors to the actual fall risk score, i.e. the second fall risk value 12. Thus, the risk factors, the weights and optionally blocks 30a to 30d belong to the risk calculator 6. Depending on the second fall risk value 12, the fall risk alert 38 is triggered (indicated by arrow 40) to the person. Besides the actual fall risk alert, further personalized fall prevention messages such as recommendations or exercises may be provided to the person. This may also depend on the second fall risk value 12.

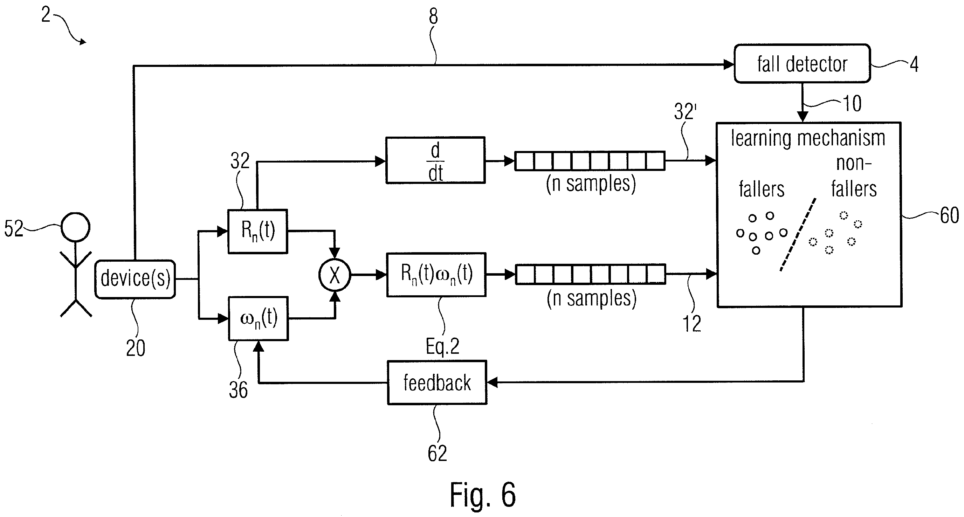

A feedback loop 42 provides the fall risk score (second fall risk value 12) back to the fall sensor 4 to adjust the sensitivity of the fall sensor e.g. to obtain less false alarms if the second fall risk value 12 is low, i.e. the probability that the person is about to fall is small. Thus, in other words, the risk calculator 6 may adjust a sensitivity of the fall sensor 4 such that an increasing fall probability indicated by the second fall risk value 10 results in a reduced probability that the fall sensor 4 misses to indicate a fall of the person. A processor (e.g. processor 26b) is configured to train the risk calculator 6 to falls of the person using the first fall risk value 10 and the set of parameters 14, if the first risk value indicates a fall of the person using the first fall risk value and related parameters of the set of parameters. The related parameters refer to (values of) the parameters of the set of parameters present at the same time when evaluating the first fall risk value to obtain training pairs especially for pre-fall patterns within the set of parameters but also for patterns at a fall or without any connection to a fall. Using these training pairs, the weights of the risk calculator may be updated (using adaptive learning). This update may be performed at predetermined times such as e.g. once a day, once a week, or once a month etc. Therefore, it is indicated that the first fall risk value is input to the weights 36 even though it would be more accurate to have the processor (e.g. Fig. 6 reveals the adaptive learning mechanism 60 for this purpose) in between. According to embodiments, the risk calculator 6 may continuously adjust the sensitivity of the fall sensor to apply a time-variant threshold to the first fall risk value 12 indicating whether the person has been falling.

This is advantageous since the update is performed as soon as a change in risk occurs. Otherwise, the sensitivity of the fall sensor would not be optimized to detect falls or to ignore non-fall events. For example, after the intake of a certain drug, the risk of falling may increase within 1-2hours. So, the sensitivity should also be updated in the same time span. An update may be required more immediately (e.g. started to rain) or in a larger time frame (e.g. due to a decline in physical capabilities). This may be referred to continuously adjusting the sensitivity, but it could be referred to as "as soon as a significant change in risk is observed".

In other words, the apparatus 2 may collect personal data from the user (i.e. the person) and context (e.g. environment) continuously, computes a time-variant second fall risk value 12 and uses this score to dynamically adapt the sensitivity of a fall detection module (i.e. fall sensor) 4 and to trigger fall prevention recommendations 38.

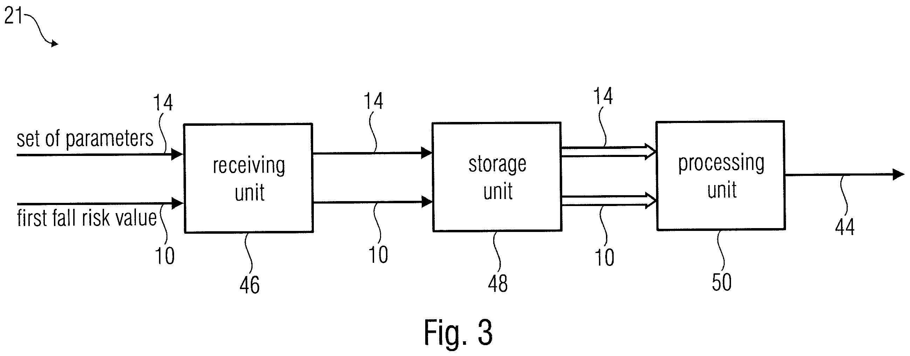

The apparatus 2 is a device or set of devices 20 with inertial sensing, positioning, storage, processing, communication capabilities and user interface (can be a smartphone, a smartwatch, an external sensor, a combination of sensors or alike), as depicted in Fig. 3 that is programmed to continuously analyze the user's movement 30a, behavior and activity 30b, health and medication 30c, environmental context 30d and automatic detection of falls 4 to identify the series of fall risk factors 32 and calculate the second fall risk value 12.

The second fall risk value (also referred to as multifactorial fall risk score) 12 may be calculated as a weighted combination 36 of multiple independent time-variant fall risk factors 32, which are a series of parameters (of the set of parameters) extracted from the continuous monitoring of the user, with basis on a background analysis of movement, activities and environmental context, as well as specific inputs provided by the user by means of questionnaires and/or tests, games, medication schedule, or deducted from user activities statistics. Environmental risk factors such as weather conditions may be additionally or alternatively extracted from the internet and each time a person falls, information on places, time and context is recorded, so that, using big data analysis techniques, environmental risks can be identified (e.g. uneven surfaces, slopes, stairs, busy streets, crowded places, poor lightening conditions, and others).

The automatic fall detection 4 may be based on 3-axial accelerometer data (and, optionally, uses also data from the gyroscope) to derive rotation angles of body segments in relation to the Earth's vertical. Thus, the fall sensor may evaluate motions of the person using values of at least one or a combination of the following sensors: gyroscope, acceleration sensor. First, a decision tree classifier (or any other suitable classifier) may be trained with data from simulated falls and non-fall daily movements. Second, the most relevant features and corresponding thresholds may be extracted and utilized e.g. in a state-machine, which organizes the signal events in fall stages events with an expected order and time-frame. Additionally, the automatic fall detection 4 may be personalized, i.e. capable of adapting the thresholds to be used according to the user's profile, physical activity level, and objectively measured fall risk 12. Moreover, if movements with a pattern similar to a fall, such as entering a car, are detected, it is possible, by combining location and previous activities information, to exclude these events from the falls classification used for the adaptive learning.

The apparatus 2 may evaluate fall risk factors against to what is considered as normal at first taking into account the typical values of the parameters encountered in the non-risk population, and pre-programmed by the apparatus. In the long term, the apparatus will adapt to the individual who is continuously using the apparatus 2. To that purpose, the evolution of each individual fall risk factor over time 12 is analyzed, so that inter-correlations between different parameters can be identified, to differentiate dependent and independent variables. By employing adaptive learning algorithms using the first fall risk value 10, the evolution of each individual independent parameter over time 32 is analyzed against the occurrence or non-occurrence of falls in a certain time period identified by the fall sensor 4.

Based on this outcome 10, the weight 36 of each fall risk factor in the combination is readjusted so that the calculated second fall risk value 12 actually characterizes the real probability of falling. Fall risk factors 32 may impact the overall second fall risk value 12 not only in the long term but also in the short term, considering rapidly-changing and highly-weighted parameters, such as those characterizing, for example, the current weather conditions, medication taken and the execution of risky activities (classification as risky depends on context).

The apparatus may also record each individual data on a server 22 (further described with respect to Fig. 3) and, using big data analysis algorithms, population data will be analyzed to infer and update 44 the list of parameters 14 and therefore the fall risk factors 32 in that e.g. the mapping rules forming a fall risk factor may be adapted, to get considered in the continuous monitoring of the users. Thus, combinations of medications triggering imbalances or associated with higher number of falls in the population may be identified.

The sensitivity of the fall detection module 4 may be adjusted according to the second fall risk value (also referred to as fall risk score) 12, i.e. the higher the risk of falling the higher the sensitivity of the fall detector.

Moreover, for battery optimization, the number of sensors used and their sampling rate may change according to the users second fall risk value 12 and the detected movements. This is why the feedback loop 42 is directed to the frame 30 instead of only to the fall sensor 4, indicating that the sensors 24 of the device are adapted according to the value of the second fall risk value 12.

If more precision is needed to analyze the signals in detail or if the user is very active, the number and sampling rate of the sensors may be increased so that movements are better perceived by the sensors 24. The fall prevention module 38 may trigger personalized, multifactorial actions for fall prevention, both in the short-term (alerts and recommendations related to the current situation - e.g. going out in rainy weather, alternative routes avoiding previously identified environmental risks, advice possible critical hours of medication side effects when the user should be more careful about balance and motor activities), medium-term (related to risks that can be changed with a specific action - e.g. advise going to the doctor to review medication or vision, adapt certain home conditions representing a risk to the person) and in the long-term (interventions related to risk factors that can only change if repeated actions are taken - i.e. exercises for balance, strength, mobility based on e.g. exergames and cognitive exercises, taken with the required frequency and intensity).

Furthermore, as already indicated, the system 21 may comprise a data server 22 connected with the device 20 (and therefore with the apparatus 2) to obtain the parameters 14 and the first fall risk value 10 from the apparatus 2. Alternatively, the second fall risk value may be omitted from being provided to the data server. In this case, the data server may obtain the unbalance of the person from the parameters 14 and to exclude false alarms based on other information and learning mechanisms. Furthermore, instead of applying an automatic or electronic update of the risk calculator, or more specifically the risk factors 32, it is also possible to monitor the evolution of parameters that are described in the literature and large population studies as being related to the risk of falling and to adapt the risk factors 32 using further knowledge from further sources such as literature or population studies.

Fig. 3 shows the data server 22 according to embodiments in more detail. The data server may comprise a receiving unit 46 configured to receive a first fall risk value 10 and a set of parameters 14 from the apparatus 2. Moreover, the data server 21 may be connected to further apparatuses to obtain further datasets comprising sets of parameters and first fall risk values. Hence, the storage unit 48 (e.g. a non-volatile storage) may store multiple first fall risk values and multiple sets of parameters from multiple apparatuses forwarded by the receiving unit. A processing unit 50 may process the multiple first fall risk values and multiple sets of parameters from multiple apparatuses (to obtain processed multiple first fall risk values and multiple sets of parameters from the multiple apparatuses and) to provide the processed multiple first fall risk values and multiple sets of parameters from the multiple apparatuses to the apparatus 2. The same information may be forwarded to the multiple apparatuses. The processing may comprise a separation of (the values of) the sets of parameters related to falls and the sets of parameters related to the absence of falls (or not related to falls) as indicated by the first fall risk value. The processed multiple first fall risk values and multiple sets of parameters may refer to the sets of parameters related to falls, which may be fed back to the apparatus by updating 44 the risk factors 32.

The risk factors may be the same for each person using any apparatus 2, wherein the weighting of the risk factors using weights 36 may be performed user specific to obtain an adapted apparatus / classifier to user-specific characteristics. Thus, the processor may be configured to process the multiple first fall risk values and the multiple sets of parameters from multiple apparatuses as to update a model for determining the first or the second fall risk factor and to provide the updated model to the apparatus to obtain an adapted fall sensor or an adapted risk calculator. The model may refer to the mapping rules applied to the parameters to obtain the risk factors. Thus, using the processor of the data server, processing power in each apparatus may be reduced. The update of the risk factors may be initiated by the apparatus 2 (e.g. at predetermined points in time such as e.g. once a day, once a week, or once a month). However, the update may be also initiated by the data server 22 if updated risk factors are available. Additionally, e.g. by adapting the risk calculator, the fall prevention recommendations may be updated if e.g. a recommendation tested on a small group of people was successful tested and approved for all people using the apparatus.

Fig. 4 shows a schematic block diagram of the device 20 according to embodiments. The basic idea has already been described with respect to Fig. 2b using the same reference numbers. The user (person) 52 is connected to the sensors 24 for sensing e.g. its acceleration using the accelerometer 24c, its 3-axis angular velocity rotations using e.g. the gyroscope 24a, its position using e.g. the GPS sensor 24b, its surrounding magnetic field using e.g. the magnetometer 24d, and/or its local atmospheric pressure using e.g. the barometer 24e. The local network module 26c may connect one or more external sensors 24f such as blood pressure sensor, scale, oximeter, sleep monitor to the device 20. Additionally, the local network module 26c enables the device 20 (and therefore the apparatus 2) to connect to the internet 54. Via internet 54, the device 22 may receive updates of the risk factors or further information such as weather forecasts or current weather conditions. Via user interface 26d, the user 52 may receive the fall prevention recommendations 38. Besides providing the fall prevention recommendations 38 to the user, the user interface 26d may receive inputs such as a medication schedule, responses to questionnaires etc.

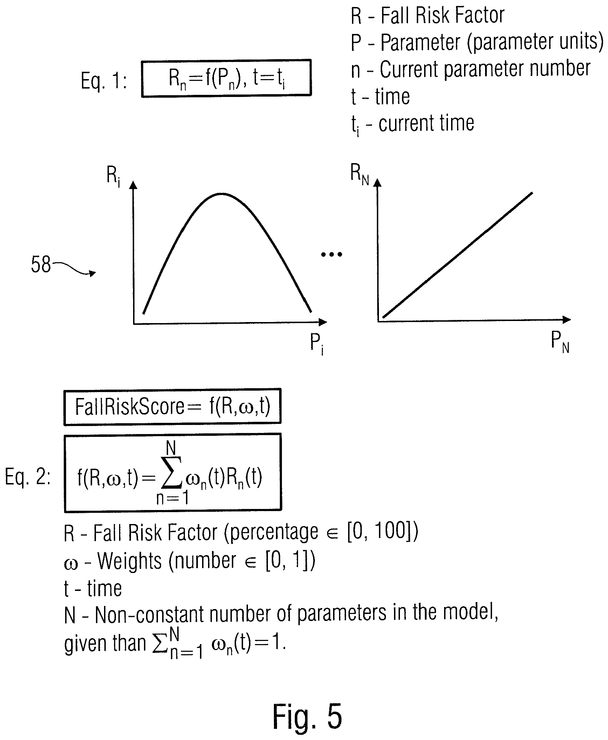

The apparatus 2 may be a smartphone or alike. The apparatus is programmed to monitor the user on a daily basis, calculate a second fall risk value that may be adapted to the evolution of the user and provide actions to prevent the occurrence of falls. In the first level, the apparatus may continuously monitor the user movements and habits (such as gait and balance capabilities, falls, medication intake, activities of daily living, sleep patterns, location information, etc.), environmental context and health. The apparatus may then map each collected parameter to a risk score, using the mapping rules in the model. A global time-variant second fall risk value is calculated as a weighted combination of each individual fall risk factor, according to Equations 1 and 2 shown in Fig. 5.

Fig. 5 shows exemplary diagrams 58 of the fall risk factor over different values of a parameter of the set of parameters. Eq. 1 reveals the mapping of parameters of the set of parameters to risks, more precisely risk factors (mapping rules). A parameter (or a combination of parameters) in the model (in the parameter units) is converted to a number between 0 and 100% that represents a fall risk factor. The conversion is conducted obeying to known mapping rules that are specified by linear or nonlinear equations. N number of rules may be used to map N number of parameters in the model. In Eq. 1 shown in Fig. 5, R is the Fall Risk Factor, P the parameter (in parameter units), n the current parameter number, t the time, and $t_i$ the current time.

Eq. 2 reveals a multivariate adaptive regression general model equation. The fall risk score (i.e. second fall risk value, both terms are used synonymously). may be calculated as a weighted sum of the individual fall risk factors. N is the number of parameters in the model. Here, R refers to the Fall Risk Factor (percentage $\in (0, 100)$), w the weights (number $\in [0, 1]$), t the time, N the Nonconstant number of parameters in the model, given than

$$\sum_{n=1}^{N} \omega_n(t) = 1 .$$

According to embodiments, an intelligent weights update mechanism based on multivariate adaptive regression techniques may be applied to allow a continuous adaptation of the overall fall risk score to the specificities of the person, considering the evolution of individual fall risk factors and their relationships against the occurrence and non-occurrence of falls in a past timeframe. A frame may be any period of time such as a day, a week, a month etc.

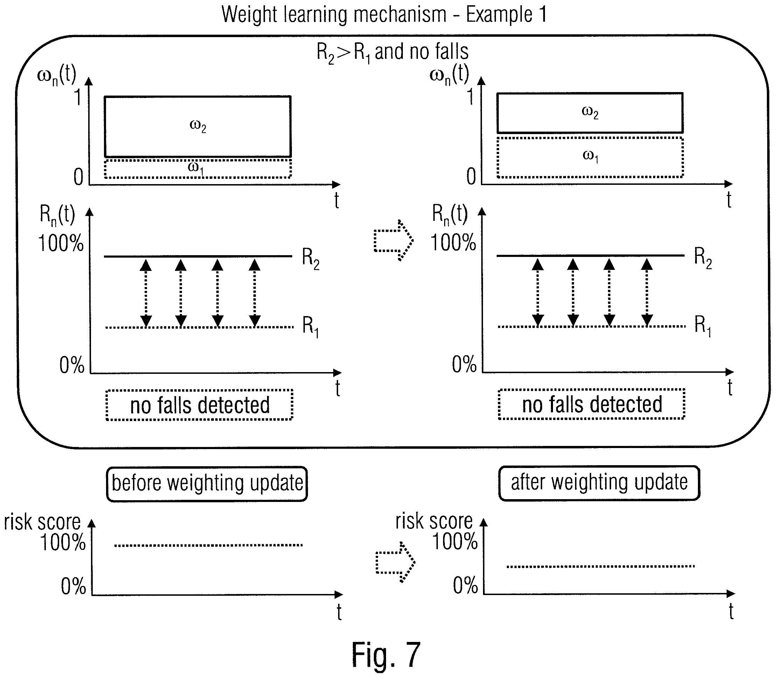

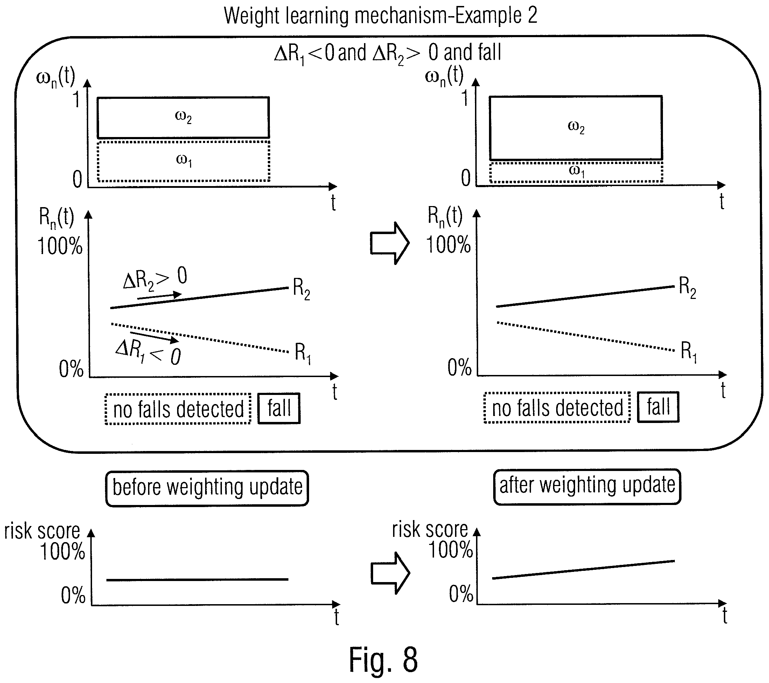

Exemplary adaptive weighting update mechanisms are described in a first example with respect to Fig. 7 and in a second example with respect to Fig. 8, wherein, in Fig. 6, a general scheme for a weights update mechanism for adaptive learning is shown.

Fig. 6 shows an individual risk factor 32 evolution (indicated by the derivative 32' of the fall risk factors 32), together with their corresponding weights 36 are analyzed against the occurrence or non-occurrence of falls (indicated by the first fall risk factor 10) during a past time period. The adaptive learning mechanism 60 will evaluate not only the evolution 32' of fall risks score 12, but also their absolute values derived from Eq. 2, so that the calculated fall risk score is in accordance with the output (i.e. occurrence or non-occurrence of falls). The learning mechanism will provide a feedback 62 on the weighting mechanism, so that it can continuously adapt to the specificities of the person. Continuously in this context may refer to the update frequency such as once a day, once a week, or once a month or any other update frequency. However, it should be pointed out that the continuous adaption is primarily referring to an adaption of the apparatus to the user over time. Furthermore, it should be pointed out that the update frequency may be context depending. For example, when a fall occurs, weights should be updated more rapidly using the learning mechanism (i.e. do not wait until e.g. the end of the week to do it). Thus, the fall sensor may trigger the adaptive learning if the first fall risk value indicates a fall of the person. Otherwise, when no falls occur, a regular update may be performed e.g. in the time frames as specified above.

This learning mechanism may be performed in the adaptive learning path indicated with reference number 10 since it carries the first fall risk value up to the learning mechanism 60. However, input to the weights may be according to Fig. 6 the feedback 62 as an output of the learning mechanism 60. The (adaptive) learning mechanism may be performed in a processor.

Fig. 7 shows the first example of the adaptive weighting update mechanism. Two risk factors (R1 and R2) were monitored during an extended time period. Each risk factor had an associated weight (w1 and w2) and w2 > w1. It was observed that R2 was higher than R1 during that time period, which resulted in a constant high global fall risk score. However, after that time frame, no falls have been detected. Considering that R1 was providing more meaningful information than R2, the weights attributed to each risk were not adapted to the individual. After applying the weight learning mechanism, weights w1 and w2 are updated, and risk score recalculated to be in accordance with its outcome (i.e. the non-occurrence of a fall). As a result the fall risk score decreases.

Fig. 8 shows the second example of the adaptive weighting update mechanism. Two risks (R1 and R2) were monitored during an extended time period. Each risk had an associated weight (w1 and w2) and w1 was equal to w2. It was observed that R2 was increasing while R1 decreased in the same time frame, which resulted in a constant global fall risk score. However, after that time frame, a fall has been detected. Considering that R2 was providing more meaningful information than R1 (R2 was actually predicting the occurrence of the fall), the weights attributed to each risk were not adapted to the individual. After applying the weight learning mechanism, weights w1 and w2 are updated, and the risk score recalculated to be in accordance with its outcome (i.e. the occurrence of a fall).

The apparatus may adjust the sensitivity of the fall detector according to the fall risk score, since a person with lower risk may be less likely to suffer an injurious fall event. The value of each fall risk factor, calculated continuously by the apparatus e.g. using the risk calculator, may, in a second level, trigger specific alerts, recommendations and exercise plans for fall prevention that will be opportunely given to the user in order to challenge him/her to be active and careful about future falls. In an implementation of the apparatus, user movements may be monitored using the inertial sensors of the mobile device (e.g. accelerometer and gyroscope). Based on activity classification algorithms running on top of the collected data, the current activity of the user may be identified and specific movement parameters, that are considered relevant in the analysis of movement quality and fall risk, are extracted. These include, but are not limited to, gait speed and steps asymmetry, number and duration of sit-to-stand transitions, level of activity and energy expended, joint range of motion, sleep quality, among others. This monitoring may also be able to identify each time a fall occurs, using e.g. the fall sensor, by employing specific fall detection algorithms based on the acceleration patterns produced by these events.

Low impact falls, unbalance or falls with no major consequences may also be considered as fall risk factors being also an input to the apparatus. The apparatus may also gather health information related to the risk of falling to characterize a user profile based on medical conditions assessment in the form of questionnaires or biometric sensors information, in case they are available and connected to the apparatus (galvanic sensors for energy expenditure, pulse oximeter for oxygen saturation estimation, heart rate sensors, humidity and temperature sensors). In other words, a parameter of the set of parameters may comprise at least one or a combination of the following information: medication of the person, humidity of the environment of the person, air pressure in the environment around the person, temperature of the environment around the person, activities of the person, current position of the person, location information of positions where other persons fell.

According to embodiments, the mobile device (the device or the apparatus) will include a user interface that will enable the user to input other relevant medical information using questionnaires and/or tests to assess medication intake, psychological state, fear of falling, vision and audition conditions. In the particular case of medication intake, the apparatus may include a medication schedule, that may be adjusted as the apparatus may learn which medications or their combination are related to imbalances and higher risk of falls. Alternatively, the apparatus or the device will be connected to a backend server and a web-based frontend that will enable the user to input the same information to the apparatus. The apparatus may also accept information from the caregivers to complete the user profile.

Besides movement, falls and health, the preferred implementation of the apparatus may be able to connect to the web to monitor environmental context such as weather conditions, to identify in case slippery floors may appear outside. Moreover, the mobile device will be able to track user's location (i.e. using GPS), so that personalized recommendations are given to the user related to the environmental context. In a preferred implementation, the apparatus may connect to a server, so that users' data related to the occurrence of falls (location, time and context) are recorded and by means of crowdsourcing external environmental factors are identified, including uneven surfaces, slopes, stairs, busy streets and crowded places, poor lightning conditions and others. In a second level, the apparatus may generate specific alerts and/or recommendations aiming to avoid the occurrence of falls, taking into account the current risks affecting the person. These triggers will be relevant in the short, medium and long term, according to the risks and time frame used to change or avoid those risks. In a preferred implementation, the apparatus will know when a certain medication (with its known side effects) is taken by the user, which, in case applied, will increase the risk of falling in the next few minutes/hours, which will then trigger alerts related to the expected side effects and what type of movements/actions a person must avoid in order not to take that risk. In other words, the apparatus may provide the person with fall prevention recommendations based on the first or the second fall risk value, wherein the fall prevention recommendations comprise one or a combination of the following recommendations: performing a medical check-up, perform a diet, following a provided nutrition plan, performing sports activities, modifying the home environment. Additionally, the apparatus may monitor the person during performing the activities and to monitor the evolution of the activities. Furthermore, the apparatus may adapt the fall prevention recommendations based on the measurement.

With time the apparatus may also learn from population data which combinations of medication will be more correlated with the occurrence of falls and in which conditions. Moreover, the apparatus will alert dangerous habits and activities and, in case the person decides to go out, advice going through routes that avoid going through areas where environmental risks were previously identified based on crowdsourced analytics of falling data and context. Therefore, the apparatus may comprise a route planer configured to determine a route (of the person) from a first position to a second position, wherein the route planer is further configured to adapt the route according the first or the second fall risk value as to avoid places where other persons had an increased probability of falling (measured e.g. from the total number of falls of any person at a certain position, e.g. considering further side effects such as rain etc. as described above to avoid this position at a certain condition characterized by a number of parameters).

In the medium term, the apparatus may opportunely do recommendations related to the advice of doing medical check-ups, diet and exercise recommendations, and home environment modifications. To improve physical capabilities related to higher risks of falling (e.g. balance, strength, flexibility), the apparatus may also trigger requests to do some specific exercises of the right type, at the appropriate times and with the appropriate frequency and intensity. In an implementation of the apparatus, these exercises may be presented to the person as exergames, to promote exercise execution and adherence to the exercises. The mobile device may be used as a sensor during exercise execution, so that movement can be analyzed and user's evolution tracked. In a preferred implementation, the mobile device may be able to connect to an external laptop or tablet that may provide visual feedback on games. More generically, the apparatus may also challenge the person to keep active, to maintain a healthy lifestyle from the beginning even if no specific risks of falling are detected. In case the apparatus identifies it as being useful, cognitive games may also be triggered.

A higher level of analysis may be made by the apparatus to find population patterns that could have influence in the risk of falling, create new fall risk factors and adapt the fall risk score of the user. Population data may be collected by multiple users using the apparatus, and may be used to identify pre-fall patterns similar to those observed in the population using big data analysis algorithms. This enables the apparatus to identify new fall risk patterns and also to trigger fall prevention actions in order to avoid falls in people presenting similar combinations of risks. The apparatus may also use population data to identify which are the most and less effective fall prevention strategies, to avoid recommending ineffective actions to people with similar combinations of risks.

According to embodiments, a fall detection module will use the second fall risk value as an input to adjust fall detection sensitivity and avoid excessive detection of false falls. The knowledge of the previous activities performed combined with the location of the user may also be considered as an input to exclude events with acceleration signatures similar to those observed in a fall event (for example, getting in the car). With this process, typical false fall alarms can be avoided. Moreover, with basis on the second fall risk value and the fall risk factors, the granularity of activity detection will change, so that more specific activities which can be considered as risky taking into account the specific factors affecting the person can be detected and more specific short-term alerts can be triggered. For example, if the apparatus detects the user has a poor balance, activities like reaching into cabinets or closet, or even gardening, can constitute a risk to the person, and therefore, should be avoided or performed with caution.

The apparatus may be defined as a framework with several modules for the analysis of the aforementioned fall risk factors, and could combine several sensors for the variety of assessments mentioned, placed in multiple locations in parallel for information combination or alone. Additionally, the apparatus compiles a display for user interaction that could be in the form of a smartphone coupled or not with a TV or a tablet. The apparatus could also work in several levels of battery consumption according to the level of precision used in the analysis of the user movements and user fall risk. In an implementation of the apparatus, the number of sensors and the rate used in signal acquisition may be lower for individuals whose fall risk score is low (meaning that the user is more active, and therefore, movement signatures are more evidently perceived in the sensor signal patterns) in favor of less battery consumption.

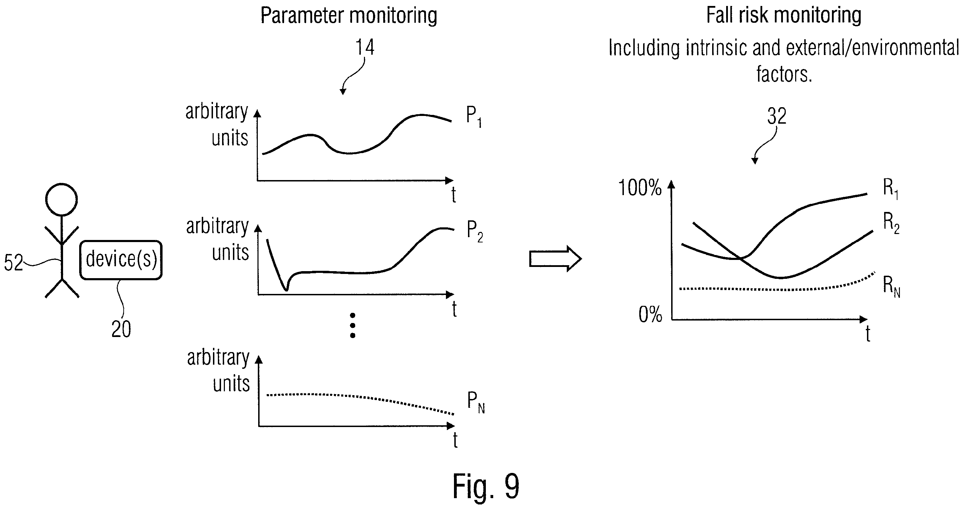

Fig. 9 shows a schematic diagram of the procedure of monitoring the parameters and to use the parameters or combine multiple parameters as risk factors. Therefore, the device (or the apparatus) will monitor multiple parameters that will then be converted into fall risks based on the known mapping rules. The history of the evolution of each individual fall risk factor is obtained. Both intrinsic (e.g. balance, strength, etc.) and external or environmental fall risk factors (e.g. medication, light humidity, etc.) may be monitored.

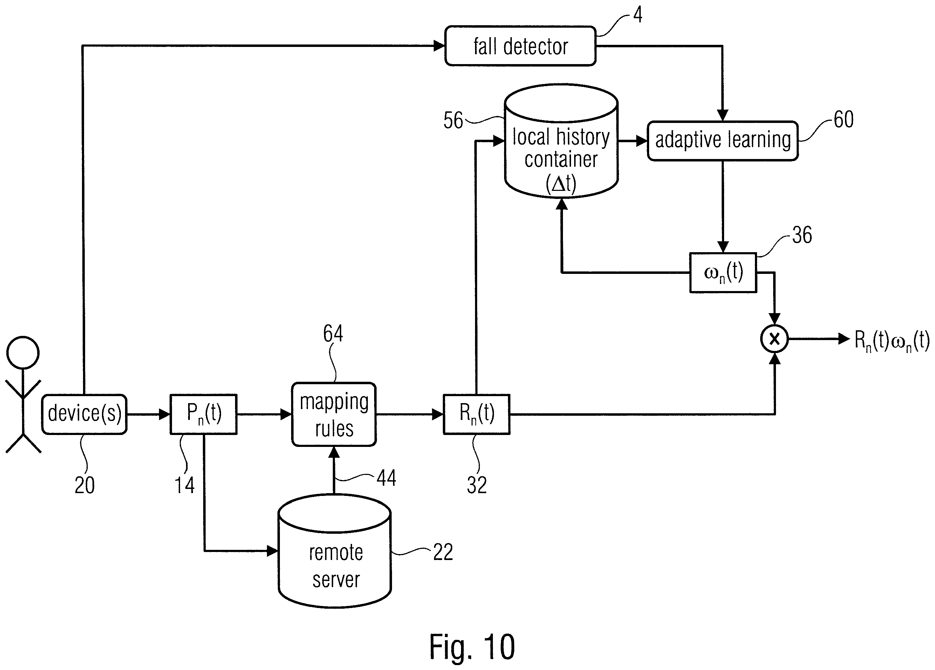

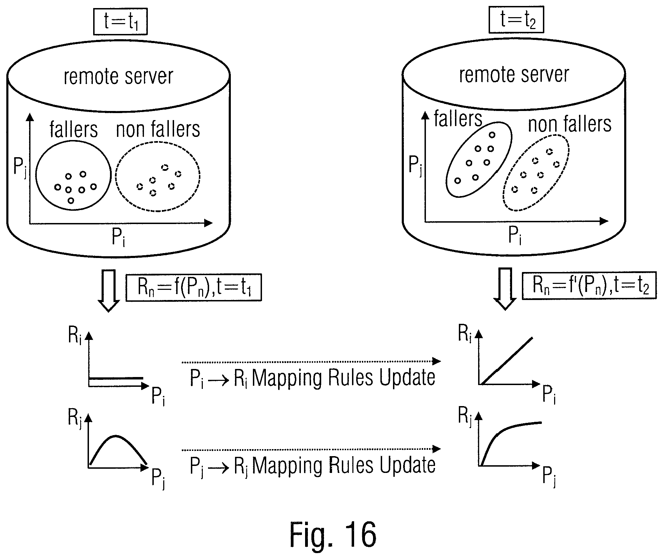

Fig. 10 shows a schematic block diagram of a general scheme of fall risk-related adaptive learning mechanisms. The device 20 or the apparatus 2 may monitor several parameters 14, including the occurrence of falls (first fall risk value). Parameters evolution and falls may be sent to a remote server 22 so that, based on crowdsource-based learning mechanisms, pre-defined mapping rules 64 can be updated 44. These mapping rules enable parameters 14 to be translated to fall risk factors 32 (more details in Fig. 16). On a first level, individual risks history evolution, as well as weights history may be stored locally e.g. in the local history container 56 (e.g. being part of the storage 26a), so that, taking into account the occurrence of falls and based on adaptive learning mechanisms 60, individual fall risk weights 36 can be adapted to the individual person (more details in Fig. 6, Fig. 7, Fig. 8). As a result, the calculated fall risk score will be more adapted to the individual person considering higher specificities, i.e. an improved prediction whether the probability of the person to fall is high.

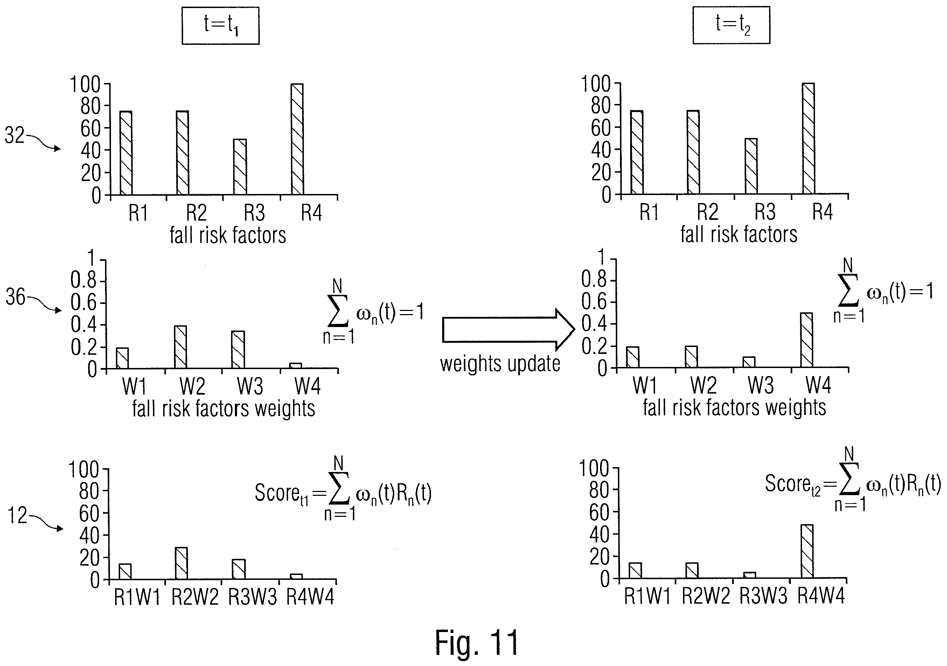

Fig. 11 shows the relation of four fall risk factors 32, the corresponding weights 36 and the resulting fall risk score 12 at two different points in time ($t_1$ and $t_2$). Fall risk factors weights are updated using adaptive learning mechanisms. As a result, fall risk score changes, adapting to the specificities of the person.

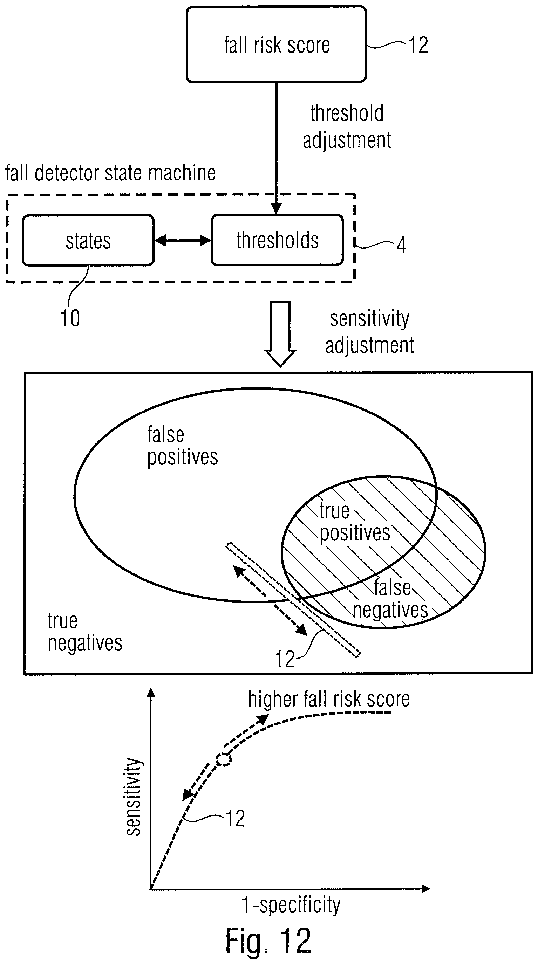

Fig. 12 shows the sensitivity adjustment of the fall sensor. According to the level of fall risk, the fall detector sensitivity may be automatically adjusted. Since for a person with higher fall risk score it is more important to detect falls, the fall detector sensitivity is increased in these cases. Fig. 12 indicates the influence of the fall risk score 12 to the fall sensor. Assuming that the fall sensor outputs a discrete first fall risk value indicating whether or not the person has been falling, the fall risk score 12 adapts an internal threshold of the fall sensor where, depending on the motion of the person, a value of the motion above the threshold may result in measuring a fall, indicated by the first fall risk value 10. However, it should be noted that the first fall risk value may be a continuous variable where the threshold to decide whether or not a fall occurred is applied in a further processing step, e.g. during the weights updating procedure.

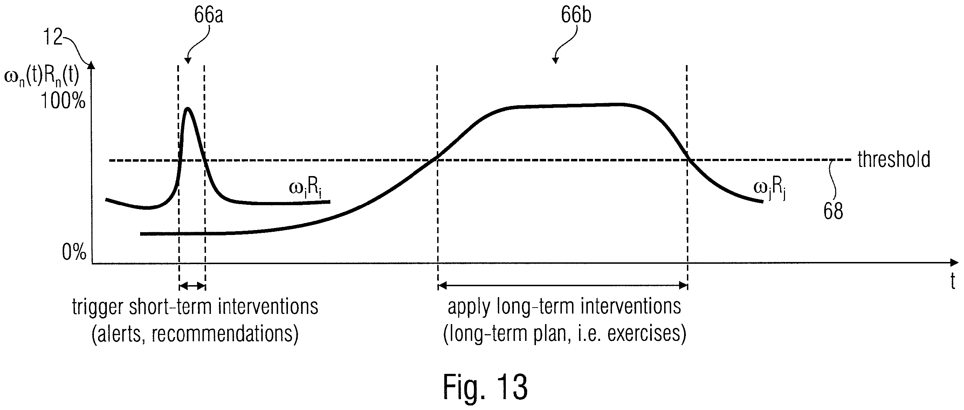

Fig. 13 shows, according to embodiments, short-term interventions and long-term interventions being optional parts of the second fall risk value, which may be applied to the individual person when an individual fall risk (factor) exceeds a certain value such as the threshold 68. Short-term fall prevention alerts and recommendations 66a may be triggered, as well as long-term interventions 66b (e.g. long-term fall prevention plan with exercises for balance, strength, mobility and cognition) considering the value and evolution of specific intrinsic and external/environmental individual fall risk factors.

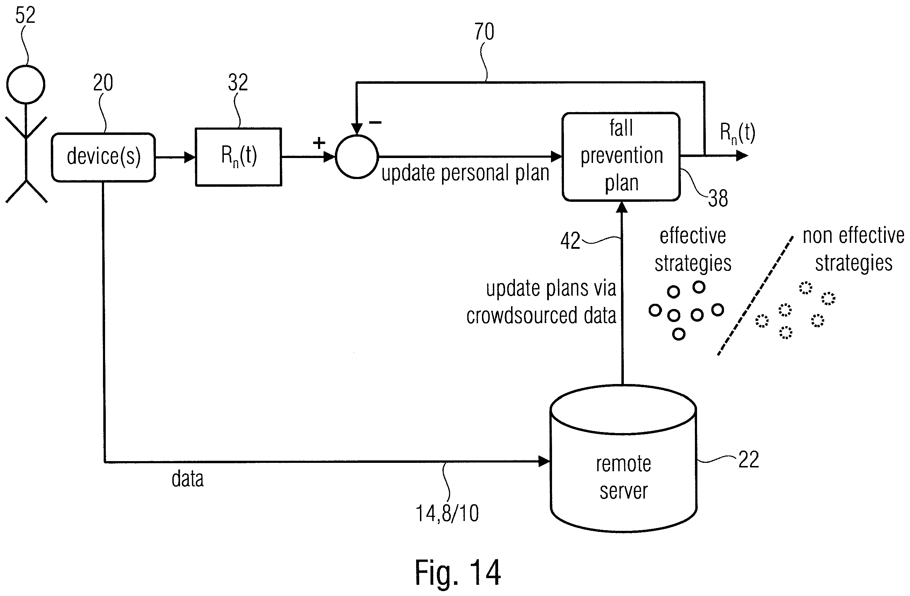

Fig. 14 shows the adaptive fall prevention mechanism of the long-term interventions according to embodiments. Individual fall risks and intervention plans executed by the person may be stored on a remote server 22, to evaluate whether the applied strategy was effective or not in its fall prevention task. Fall prevention plans as part of the fall prevention recommendations 38 may be continuously improved (using e.g. feedback loop 70) considering the data gathered via crowdsourcing, e.g. from the remote server 22.

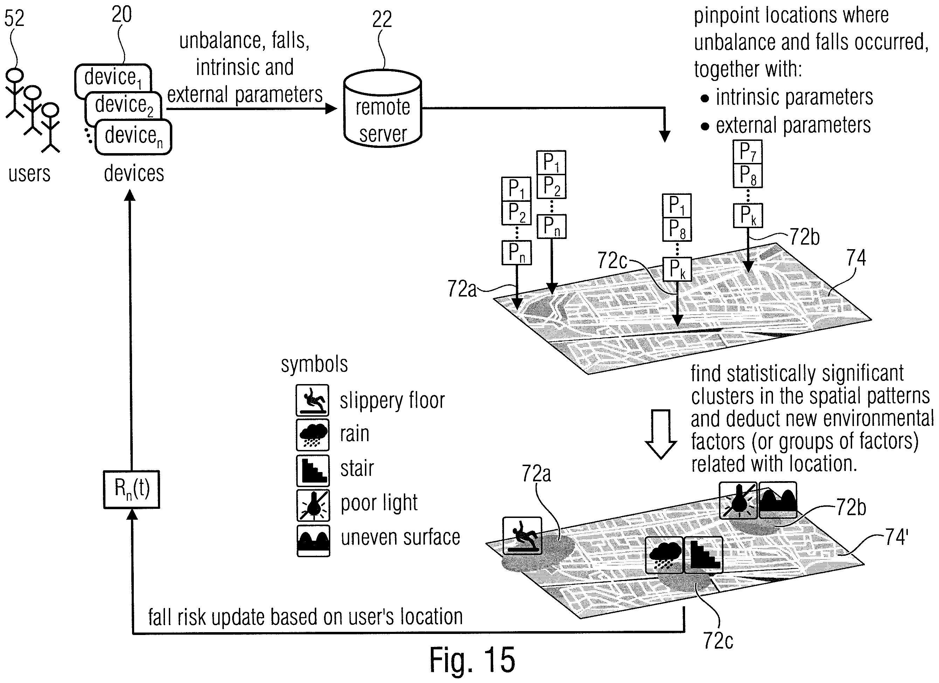

Fig. 15 shows embodiments of the crowdsourcing approach. While some locations 72a, 72b, 72c are dangerous due to architectural characteristics (e.g. due to floor characteristics (cf. e.g. stairs in 72c) or changes in height (cf. e.g. uneven surface in 72b)), other locations represent higher risks of falling just when certain environmental conditions are measured (e.g., stairs can be become dangerous when it's raining (cf. 72a); uneven surfaces become more dangerous when there is poor lightning conditions (cf. 72b)). Thus, the device 20, or more generally the apparatus 2 of multiple persons 52 may provide location information about falls together with environmental conditions at the time the fall occurred to the remote server 22. This information may refer to an unbalance of the persons, falls and/or intrinsic and/or external parameters. These locations may be evaluated, and e.g. as Fig. 15 illustrates, depicted on a map 74 based on e.g. the respective GPS location. The single locations may be grouped to form areas that show a higher risk of falling, as indicated on map 74'. This information may be further input to the risk factor calculation as an external parameter to adapt the value of the fall risk score. According to embodiments, the apparatus 2 comprises a route planer configured to determine a route from a first position to a second position, wherein the route planer is further configured to adapt the route according the second fall risk value or based on the location information of multiple persons derived from the remote server 22. It is e.g. possible, that a person whose fall risk factor (second fall risk value) is very low, i.e. the probability of falling is very low, such that this person may be guided through the areas 72a-c with a higher probability of falling regardless the higher probability of falling, since it is still not likely that the person falls. However, besides this consideration, it is also possible to omit guiding a person to the areas of higher probability of falling anyway without considering the second fall risk value.

Hence, in general, the apparatus may obtain first fall risk values and sets of parameters from further persons different from the person and to adapt a determination of the first or the second fall risk value based on the first fall risk values from the further persons and respective parameters of the sets of parameters from the further persons. This may be referred to as crowdsourcing.

Fig. 16 shows schematic representations of the mapping rules update at from a first point in time $t_1$ to a second point in time $t_2$, Mapping rules specify how a certain parameter is converted to a fall risk factor (e.g. a number between 0 and 100%). These optionally linear or non-linear rules are learned from population data that is stored in a remote server. Crowdsource-based learning may be applied to learn or update existing mapping rules, based on the occurrence of falls.

Following are listed a number of publications related to the topic of this disclosure. None of the publications reveal that a fall sensors sensitivity is adjusted by a feedback loop of a (first) fall risk value, wherein the (first) fall risk value itself is influenced by the output of the fall sensor. In other words, the publications do not show a risk calculator configured to determine a second fall risk value based on a set of parameters, wherein a sensitivity of the fall sensor is adjusted using the second fall risk value. Further differences to the publications are described below.

US 7612681 B2 shows a system and method for predicting fall risk for a resident. However, embodiments of the apparatus and method described in the present disclosure go beyond this data collection of the US patent by proposing adaptive learning algorithms, a time-variant weighted combination of multiple fall risk factors. Moreover, the disclosure proposes to obtain fall risk factors not only from sensors attached to the user's body but also from several different sources, such as profile data or crowdsourced information of hazardous locations and considering short-term variations of factors, such as environmental context, medication taken and execution of risky activities. Furthermore, embodiments of the present disclosure present a combined solution for a multifactorial assessment of fall risk, optionally implemented on a continuous, adaptive and/or real-time basis.

WO 2009156936 A2 shows a system and method for determining a personal health related risk. In contrast, embodiments of the present disclosures show a multifactorial assessment of fall risk, optionally implemented on a continuous, adaptive and/or real-time basis.

US 2013023798 A shows a method for body-worn sensor based prospective evaluation of falls risk in community-dwelling elderly adults. However, only parameters extracted from the TUG (Timed-Up-and-Go) test are used in the model. Embodiments of the present disclosure go beyond this, by proposing the calculation of a fall risk score based on multifactorial data collected continuously while the system is being used. Furthermore, adaptive mechanisms may be applied to improve calculations and individualize fall risk score calculation based on detected falls, by means of changing individual fall risk weights in the original fall risk model.

US 2013303860 A shows a system and methods for use in fall risk assessment. However, embodiments of the present disclosure provide a personal adaptive mechanism to improve the quality of the outputs to the user, such as the fall prevention recommendations.

EP 2329470 B1 shows a fall detection and/or prevention system. In contrast to embodiments of the present disclosure where the fall sensor (measuring actual falls) is adapted based on the fall risk score being a measure of how likely it is that the person is about to fall, wherein the cited European patent proposes to adapt and update the algorithms based on the occurrence of falls, not a fall risk score. Thus, in contrast to the present disclosure, this is not a solution for minimizing false positive detections in the case of non-occurrence of falls for very long periods of time.

WO 2015036245 A1 shows a fall detection system and method. In contrast, embodiments of this disclosure show a fall risk index computed from several population risk factors, obtained for each person from sensor information and other sources.

US 7541934 B2 shows a Method and device for fall prevention and detection, from Secu-management B.V.. In contrast, according to embodiments, the user is alerted by the device in the short-term but the alerts are triggered upon the detection of potentially risky situations and contexts (identified from crowdsourced data) combined with the fall risk level of the user. Moreover, long-term actions towards fall prevention may be also triggered and adapted based on the evolution of the risk of falling.

US 20100124737 A1 shows a method and system for fall prevention in older adults. In contrast, this disclosure shows embodiments where the apparatus personalizes actions for fall prevention automatically, based on the monitored parameter. Said actions may include not only alerts and recommendations. but also exercise interventions. which are monitored using the mobile device.

WO 2014136112 A1 shows a patient-specific rehabilitative video game. In contrast, the apparatus according to embodiments may be setup automatically based on the specificities of the person (which are also perceived by the apparatus) and the evolution of the fall risk factors over time.

"iStand Falls Prevention®" is a solution based on video modules that leads the user through a number of exercises clinically proven to reduce the risk of falls among seniors.

In contrast, embodiments show the apparatus being distinctive since while exercise interventions are based on clinical evidence, a detailed plan is constructed for each user according to the risk of falling and most prominent risk factors and this plan may be adjusted as the user evolves. Moreover, the compliance to the exercise plan may be monitored using a mobile device with a set of sensors in order to assure that the exercises are performed correctly and retrieve data that will contribute to assess the evolution of the user.

The Project 'iStoppFalls' has been developed within the European Commission's Seventh Framework Program (FP7) aiming to create an Information and Communication Technology based system to regularly assess a person's risk of falling in their own home and to deliver an individual and tailored homebased exercise and education program for fall prevention. The two following publications describe parts of the system.

Y. J. Gschwind, S. Eichberg, H. R. Marston, A. Ejupi, H. de Rosario, M. Kroll, M. Drobics, J. Annegarn, R. Wieching, S. R. Lord, K. Aal and K. Delbaere, "ICT-based system to predict and prevent falls (iStoppFalls): study protocol for an international multicenter randomized controlled trial," BMC Geriatr., vol. 14, no. 1, p. 91, Aug. 2014. In contrast, embodiments of the present disclosure show to create an intervention plan for each user in which the exercises and recommendations chosen depend on the risk of falling and most prominent risk factors. This plan may be adjusted automatically as the user evolves, not only in response to the correct execution of the exercises, but also to the evolution of the fall risk score and fall risk factors. Moreover, this adjustment of the intervention plan may be based on altering the duration, level of difficulty, number of repetitions, as well as by automatically proposing different types of exercises.

Hilde AE Geraedts, Wiebren Zijlstra, Wei Zhang, Sjoerd Bulstra and Martin Stevens, "Adherence to and effectiveness of an individually tailored home-based exercise program for frail older adults, driven by mobility monitoring: design of a prospective cohort study", BMC Public Health, vol. 14, p. 570, 2014. In contrast, embodiments of the present disclosure show that the prescription of the exercises may be set automatically by the system and also learns with the evolution of the user in terms of fall risk score and fall risk factors.

US 2015148113 A shows patient-specific rehabilitative video games. In contrast, embodiments of the present disclosure relate to therapy plans being learned from multiple users based on big data analysis.

US 2015260514 A shows method to determine physical properties of the ground. In contrast, embodiments of the present disclosure show the apparatus using crowdsourcing techniques.

US 8082122 B2 show a mobile device having a motion detector. In contrast, embodiments of the present disclosure show adjusting the sensors sampling rate and/or also the number of sensors, based on the fall risk score.

A. Weiss, M. Brozgol, M. Dorfman, T. Herman, S. Shema, N. Giladi and J. M. Hausdorff, "Does the Evaluation of Gait Quality During Daily Life Provide Insight Into Fall Risk? A Novel Approach Using 3-Day Accelerometer Recordings", Neurorehabilitation and Neural Repair, vol. 27(8), p. 742, 2013. In contrast, embodiments of the present disclosure show that a set of parameters derived from the combination of one or more sensors and used to determine a second fall risk value are adapted by an adaption unit.

Embodiments of the present disclosure relate to an apparatus or a method that dynamically extracts information from data obtained from the normal daily activity of the user and his environment, have the ability to learn and to deduct new knowledge automatically from the data, and uses this information and knowledge to compute the risk of falling and suggest short and long-term interventions for falls prevention that are continuously adapted to the evolution of the user.

Embodiments of the present disclosure target community-dwelling, independent, seniors and pre-seniors who have low or high fall risk scores. Other applications may be directed to younger users that may be at risk of falling (due to certain pathologies, for example), institutionalized patients who may be at risk of falling. The apparatus may be adapted to other conditions in which specific monitoring and intervention are used, such as training of high performance athletes, high risk professionals (security and safety, firefighters), patients needing rehabilitation, individuals that need to control their weight (obesity, diabetes patients), monitoring children, mostly to alert when a fall event occurs and monitor his/her progress in terms of equilibrium and strength.

Such a system is suitable to be distributed by mobile network operators, for example, as it would induce a more frequent usage of smartphones by the 65 + segment of the population. Smartphone and wearables manufactures may also have commercial interest in such a solution, since it could be integrated in their products at the production level. These products would be highly differentiated in the market and address the needs of a growing market segment with increasing purchasing power and interest in technology. Rehabilitation or other healthcare provider units may also be potential commercial partners, benefiting from the referred advantages as the deployment of such a system in their facilities would allow a more cost-effective care delivery.

The present disclosure further relates to embodiments described by the following examples. The examples may be directed to the apparatus and method as claimed.

A first example of the present disclosure relates to a method to compute the fall risk score taking into account personal and environmental data and their evolution in the short and long term, the method comprising: continuous collection of user data for the background analysis of movement, activities, falls, health and environmental context, relying on the analysis of sensor data, and inputs provided by the user by means of questionnaires, medication schedules, tests and games; fall risk score calculated as time-variant weighted combination of multiple fall risk factors; fall risk score considering short-term variations of factors (such as environmental context, medication taken and execution of risky activities); adaptive learning algorithms, that will analyze the evolution of each individual risk factor over time against the occurrence or non-occurrence of falls in a time period, in order to adapt the system (weights of the risk factors) to the specificities of the person.

This is advantageous, since the risk of falling is continuously and automatically screened without direct intervention of the user or trained professionals. This feature provides a closer monitoring of the user without representing a burden and time consuming tasks to either the user or the healthcare system. Multiple aspects contributing to the risk of falling are measured and combined taking into account their relative importance to each user. It enables a more thorough analysis of several fall risk factors and a better representation of the risk of falling than currently done with state-of-the-art tests performed at healthcare facilities. Short-term variations of fall risk factors are never taken into account in the state-of-the-art tests performed at healthcare facilities, but monitoring these variations enables to trigger short-term alerts and recommendations to prevent falls right before they are about to happen.

According to a second example related to the first example, the method comprising adjusting the sensitivity of the fall detection based on the fall risk score.

Automatic technology-based fall detectors usually suffer from the frequent occurrence of false fall alarms that disturb the users and cause lack of adherence to these systems. By adjusting the sensitivity of the fall detection automatically with basis on the continuously adapting fall risk score the false alarms may be minimized when the risk of falling is low, while making sure that no real fall is missed when the risk is high. This is extremely useful as low risk users tend to be more active and thus perform more activities that are likely to trigger more false alarms. Thus, it is advantageous to decrease false fall detection alarms and consequently improve the adherence to fall detection systems.

According to a third example related to the second or the first example, the method comprises triggering short-term fall prevention alerts and recommendations, as well as long-term interventions (e.g. long-term fall prevention plan with exercises for balance, strength, mobility and cognition) considering the value and evolution of specific intrinsic and external/environmental individual fall risk factors.

This is advantageous, since fall prevention alerts that are based on the value of specific fall risk factors are more precisely directed to the cause of the risk of falling and therefore, may more effectively prevent falls from happening.

According to a fourth example related to any of the first to the third example, the method comprises continuous learning of effective fall prevention strategies based on big data analysis of effective and non-effective plans versus fall risks evolution, wherein new effective fall prevention activities can also be learned considering unexpected changes in life-styles and habits (i.e. changes that were not advised by the system) that result in consistent changes in individual fall risk scores.

This is advantageous, since long-term fall prevention plans that are precisely tailored to fall risk factors of each user that evolve over time as these factors progress are more effective as fall prevention strategies and allow to minimize or revert the identified fall risk factors and ultimately avoid falls. The apparatus may be adapted according to the evolution of the person, which allows a continuous and specific solution to prevent the occurrence of falls. Ultimately the system will provide a more efficient solution when compared to static fall prevention programs.

According to a fifth example related to any of the first to the fourth example, the method comprises crowdsourcing of locations of frequent falls, associated with a time of occurrence and context (weather conditions and surface characteristics,) to deduct external/environmental factors (e.g. locations of uneven surfaces, slopes, stairs, busy streets, slippery floors, etc).

This is advantageous, since mapping of the most dangerous locations and conditions to the occurrence of falls allows to generate alerts when a user who has already an intrinsic risk of falling is approaching these locations or is outside under certain conditions. These short-term alerts may prevent falls right before they are about to happen.

According to a sixth example related to any of the first to the fifth example, the method comprises inferring new fall risk factors and update parameters-risks mapping rules automatically from the continuously growing population data taking into account the correlation of the parameters monitored with the falls detected by the system.

This is advantageous, since the apparatus should have predicting capability to infer new fall risks and prevention strategies automatically, this will allow more effectively reverting fall risk factors over time. The apparatus may also learn with the users in a way that it could take advantage of the population data to adapt the prescribed exercise program based on success rates. By being automatically incorporating knowledge generated from population observations, the system will be continuously updated according to the evidence and thus ensure maximum efficiency in falls prevention.

According to a seventh example related to any of the first to the sixth example, the method comprises trading-off battery drain and detection accuracy during continuous monitoring by adjusting the number of sensors used and/or sensor sampling rate according to the fall risk score. In other words, the risk calculator may receive the set of parameters and adapt a frequency of an update of the set of parameters based on the second fall risk value. Accordingly, the fall sensor may receive the set of parameters and adapt a frequency of an update of the motion (i.e. parameters directed to the motion of the person such as information of a gyroscope or accelerometer etc.) of the person based on the second fall risk value.

Automatic technology-based Activity Trackers usually imply a fast battery drain of the device in which they are implemented and this causes lack of adherence to these systems. Thus, it is advantageous to increase the battery life time, since it will increase the overall acceptance of the apparatus. By adjusting the sensor sampling rate, more granularity can be provided for the monitoring algorithms, according to the level of precision that may be set based on the fall risk score. Adjusting the number of sensors used for continuous monitoring may also provide a more tailored result to the user, since new sources of information can increase the performance level when needed. Automatic management between battery drain and detection accuracy will ensure that the system always fit the user's needs.

According to an eighth example related to any of the first to the seventh example, the method comprises refining granularity of activity detection based on the fall risk score and fall risks, so that more specific short-term alerts can be triggered.

This is advantageous, since, when the fall risk score is high or a certain risk factor is detected, more subsets of activities may be detected, for example risky movements, so that more precise short term alerts may be triggered. In other circumstances, this activity detection granularity may be reduced in order to minimize battery drain.

According to a ninth example related to any of the first to the eighth example, the method comprises detecting low impact falls, unbalance or falls with no major consequences as risk factors.

This is advantageous, since it improves the accuracy of the fall risk score.

According to a tenth example related to any of the first to the ninth example, the method comprises understanding events with acceleration signatures similar to those observed in a fall event as non-falls based on the knowledge of the previous activities performed combined with the location of user (for example, getting in the car).

This also helps minimizing the occurrence of false fall alarms and thus improving adherence to fall detection systems. The major advantage is its ability to learn and adjust based on the collected data and information generated both at the level of the user and also the population. These adjustments are reflected in more accurate and personalized fall risk score calculation and identification of fall risk factors, as well as in more effective fall prevention strategies, while maximizing adherence by minimizing false alarms and battery drain.

According to a eleventh example related to any of the first to the tenth example, all the above mentioned method steps can be implemented in a single device.

According to a twelfth example, the fall sensor is configured to evaluate motions of the person using values of at least one or a combination of the following sensors: accelerometer, gyroscope, magnetometer, barometer, image and/or sound.

According to a thirteenth example, the risk calculator is configured to use a parameter of the set of parameters comprising at least one or a combination of the following information: medication of the person, humidity of the environment of the person, air pressure in the environment around the person, temperature of the environment around the person, activities of the person, current position of the person, location information of positions where other persons fell, health status, physical capability (e.g. strength, flexibility, balance, gait velocity, movement symmetry, etc.).

According to a fourteenth example, the apparatus is configured to provide the person with fall prevention recommendations based on the first or the second fall risk value, wherein the fall prevention recommendations comprise one or a combination of the following recommendations: performing a medical check-up, perform a diet, following a provided nutrition plan, performing sports activities, performing specific exercises for fall prevention based on the specific encountered risks factors, modify the home environment, avoid certain routes, avoid walking outdoors in rainy weather, avoid the execution of certain activities at certain time periods of the day (e.g. after taking a medicine, etc.).

According to a fifteenth example, the apparatus is configured to monitor the person during performing the activities and to monitor the evolution of the activities.

According to a sixteenth example, the apparatus is configured to trigger the fall risk alert if the first fall risk value indicates that the person is about to fall and wherein the sensitivity of the fall sensor is adjusted based on the second fall risk value to change a threshold of the first fall risk value, wherein the threshold indicates that the person is about to fall.

According to a seventeenth example, the risk calculator is configured to continuously adjust the sensitivity of the fall sensor to apply a time-variant threshold to the first fall risk value indicating if the person has been falling.

Although the present invention has been described in the context of block diagrams where the blocks represent actual or logical hardware components, the present invention can also be implemented by a computer-implemented method. In the latter case, the blocks represent corresponding method steps where these steps stand for the functionalities performed by corresponding logical or physical hardware blocks.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus. Some or all of the method steps may be executed by (or using) a hardware apparatus, like for example, a microprocessor, a programmable computer or an electronic circuit. In some embodiments, one or more of the most important method steps may be executed by such an apparatus.

The inventive transmitted or encoded signal can be stored on a digital storage medium or can be transmitted on a transmission medium such as a wireless transmission medium or a wired transmission medium such as the Internet.

Depending on certain implementation requirements, embodiments of the invention can be implemented in hardware or in software. The implementation can be performed using a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the inventive method is, therefore, a computer program having a program code for performing one of the methods described herein, when the computer program runs on a computer.

A further embodiment of the inventive method is, therefore, a data carrier (or a non-transitory storage medium such as a digital storage medium, or a computer-readable medium) comprising, recorded thereon, the computer program for performing one of the methods described herein. The data carrier, the digital storage medium or the recorded medium are typically tangible and/or non-transitory.

A further embodiment of the invention method is, therefore, a data stream or a sequence of signals representing the computer program for performing one of the methods described herein. The data stream or the sequence of signals may, for example, be configured to be transferred via a data communication connection, for example, via the Internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

The above described embodiments are merely illustrative for the principles of the present invention. It is understood that modifications and variations of the arrangements and the details described herein will be apparent to others skilled in the art. It is the intent, therefore, to be limited only by the scope of the impending patent claims and not by the specific details presented by way of description and explanation of the embodiments herein.

**Claims**

1. Apparatus (2) for triggering a fall risk alert (38) to a person, the apparatus comprising:

    a fall sensor (4) configured to evaluate motions of the person so as to obtain a first fall risk value (10);
    a risk calculator (6) configured to determine a second fall risk value (12) based on a set of parameters,
    an adaption unit (15) configured to adapt a weight (36) associated with a parameter of the set of parameters (14) as to determine the second fall risk value (12) based on the first fall risk value (10); and
    wherein the apparatus (2) is configured to trigger the fall risk alert (38) depending on the second fall risk value.

2. Apparatus according to claim 1, wherein a sensitivity of the fall sensor is adjusted using the second fall risk value.

3. Apparatus (2) for triggering a fall risk alert to a person, the apparatus comprising:

    a fall sensor (4) configured to evaluate motions of the person so as to obtain a first fall risk value;
    a risk calculator (6) configured to determine a second fall risk value based on a set of parameters, wherein a sensitivity of the fall sensor is adjusted using the second fall risk value; and
    wherein the apparatus is configured to trigger the fall risk alert depending on the first and the second fall risk value.

4. Apparatus according to claim 3, wherein the apparatus comprises a adaption unit configured to adapt a weight for a parameter of the set of parameters as to determine the second fall risk value based on the first fall risk value; and
    wherein the apparatus is configured to trigger the fall risk alert depending on the first and the second fall risk value by triggering the fall risk alert based on the second fall risk value adapted by the first fall risk value.

5. Apparatus according to any of the preceding claims, wherein the apparatus is configured to trigger the fall risk alert (38) comprising recommendations for the user to prevent future falls dependent on the second fall risk value.

6. Apparatus according to any of claims 2 to 5, wherein the risk calculator is configured to adjust the sensitivity of the fall sensor such that an increasing fall probability indicated by the second fall risk value results in a reduced probability that the fall sensor misses to indicate a fall of the person;
    wherein a processor is configured to train the risk calculator to falls of the person using the first fall risk value and the set of parameters, if the first risk value indicates a fall of the person using the first fall risk value and related parameters of the set of parameters.

7. Apparatus according to any of the preceding claims, wherein the risk calculator is configured to determine a trend within consecutive values of parameters of the set of parameters and to trigger the fall risk alert comprising fall prevention recommendations if the trend within the consecutive values of the parameters indicates an increasing fall risk probability; or
    trigger the fall risk alert if one parameter or combination of parameters of the set of parameters exceeds a threshold indicating an increasing fall risk probability.

8. Apparatus according to any of the preceding claims, wherein the apparatus is configured to obtain first fall risk values and sets of parameters from further persons different from the person and to adapt the determination of the first or the second fall risk value based on the first fall risk values from the further persons and respective parameters of the sets of parameters from the further persons.

9. Apparatus according to any of the preceding claims comprising a route planer configured to determine a route from a first position to a second position, wherein the route planer is further configured to obtain regions with a higher probability of falling and configured to adapt the route according the higher probability of falling or according to the second fall risk value.

10. Apparatus according to any of the preceding claims, wherein the risk calculator is configured to receive the set of parameters and to adapt a frequency of an update of the set of parameters based on the second fall risk value.

11. Data server (21) comprising:

a receiving unit configured to receive first fall risk values and sets of parameters from an apparatus according to any of claims 1 to 10; a storage unit configured to store multiple first fall risk values and multiple sets of parameters from multiple apparatuses; a processor configured to process the multiple first fall risk values and multiple sets of parameters from multiple apparatuses and to provide the processed multiple first fall risk values and multiple sets of parameters from multiple apparatuses to the apparatus.

12. Data server according to claim 11, wherein the processor is configured to process the multiple first fall risk values and the multiple sets of parameters from multiple apparatuses so as to update a model for determining the first or the second fall risk value and to provide the updated model to the apparatus to obtain an adapted fall sensor or an adapted risk calculator.

13. Method for triggering a fall risk alert to a person, the method comprising:

evaluating motions of the person so as to obtain a first fall risk value; determining a second fall risk value based on a set of parameters, wherein a sensitivity of the fall sensor is adjusted using the second fall risk value; and triggering a fall risk alert depending on the first and the second fall risk value.

14. Method for triggering a fall risk alert to a person, the apparatus comprising:

evaluating motions of the person so as to obtain a first fall risk value; determining a second fall risk value based on a set of parameters; adapting a weight for a parameter of the set of parameters as to determine the second fall risk value based on the first fall risk value; and triggering the fall risk alert depending on the second fall risk value.

15. Method comprising:

receiving first fall risk values and sets of parameters from an apparatus according to any of claims 1 to 10; storing multiple first fall risk values and multiple sets of parameters from multiple apparatuses; processing the multiple first fall risk values and multiple sets of parameters from multiple apparatuses and providing the processed multiple first fall risk values and multiple sets of parameters from multiple apparatuses to the apparatus.

16. Computer program for performing the methods of any of claims 13 to 15

2

4

8

motion → | fall sensor |

second fall risk value  10   first fall risk value

12

set of parameters  14  → | risk calculator |  → second fall risk value  12

6

## Fig. 1a

2

4

8

motion → | fall sensor |  → first fall risk value  10

second fall risk value  12

set of parameters  14  → | risk calculator |

6

## Fig. 1b

Fig. 1c

Fig. 1d

Fig. 2a

EP 3 346 402 A1

Fig. 2b

Fig. 3

EP 3 346 402 A1

Fig. 4

EP 3 346 402 A1

R - Fall Risk Factor
P - Parameter (parameter units)
n - Current parameter number
t - time
$t_i$ - current time

Eq. 1: $\boxed{R_n = f(P_n),\ t = t_i}$

$FallRiskScore = f(R, \omega, t)$

Eq. 2: $\boxed{f(R, \omega, t) = \sum_{n=1}^{N} \omega_n(t) R_n(t)}$

R - Fall Risk Factor (percentage $\in [0, 100]$)
$\omega$ - Weights (number $\in [0, 1]$)
t - time
N - Non-constant number of parameters in the model,
given than $\sum_{n=1}^{N} \omega_n(t) = 1$.

## Fig. 5

Fig. 6

EP 3 346 402 A1

Weight learning mechanism - Example 1

Fig. 7

EP 3 346 402 A1

Weight learning mechanism-Example 2

Fig. 8

Parameter monitoring

14

Fall risk monitoring

Including intrinsic and external/environmental factors.

32

Fig. 9

EP 3 346 402 A1

Fig. 10

EP 3 346 402 A1

Fig. 11

fall risk score ~12

threshold
adjustment

fall detector state machine

states ↔ thresholds ~4

10

sensitivity
adjustment

false
positives

true
positives

false
negatives

true
negatives

12

higher fall risk score

sensitivity

12

1-specificity

Fig. 12

Fig. 13

EP 3 346 402 A1

Fig. 14

EP 3 346 402 A1

Fig. 15

Fig. 16

Europäisches
Patentamt

European
Patent Office

Office européen
des brevets

**EUROPEAN SEARCH REPORT**

Application Number

EP 17 15 0270

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/220153 A1 (ANNEGARN JANNEKE [NL] ET AL) 4 August 2016 (2016-08-04) | 3,13,16 | INV.<br>G06F19/00 |
| Y | * paragraph [0004] - paragraph [0030] *<br>* paragraph [0040] *<br>* paragraph [0062] - paragraph [0067] *<br>* paragraph [0079] - paragraph [0096] *<br>* paragraph [0113] - paragraph [0114] *<br>* figures 5,6,8 * | 1,2,<br>4-10,14 | ADD.<br>A61B5/11<br>G08B21/04 |
| X | GB 2 352 815 A (CAMERON KEITH HENDERSON [GB]) 7 February 2001 (2001-02-07) | 11,12,15 | |
| Y | * page 1, paragraphs 1, 3 *<br>* page 2, paragraph 1 - paragraph 2 *<br>* page 3, paragraph 2 - paragraph 4 *<br>* page 4, paragraph 1 - page 5, paragraph 1 *<br>* figure 1 *<br>* table 1 *<br>* claims 11,19,23 * | 1,2,<br>4-10,14 | |
| A | US 2011/295621 A1 (FAROOQ FAISAL [US] ET AL) 1 December 2011 (2011-12-01)<br>* paragraph [0022] *<br>* the whole document *<br>* paragraph [0004] *<br>* paragraph [0066] *<br>* paragraph [0060] *<br>* paragraph [0066] * | 1-16 | **TECHNICAL FIELDS SEARCHED (IPC)**<br><br>G06F<br>A61B<br>G08B |
| A | US 2016/147959 A1 (MARIOTTINI GIAN-LUCA [US] ET AL) 26 May 2016 (2016-05-26)<br>* paragraph [0005] - paragraph [0007] *<br>* paragraph [0022] - paragraph [0023] *<br>* paragraph [0035] - paragraph [0054] *<br>* paragraph [0072] * | 1-16 | |

-/--

The present search report has been drawn up for all claims

| Place of search | Date of completion of the search | Examiner |
|---|---|---|
| Munich | 7 June 2017 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 17 15 0270

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BOURKE ET AL: "A threshold-based fall-detection algorithm using a bi-axial gyroscope sensor", MEDICAL ENGINEERING & PHYSICS, BUTTERWORTH-HEINEMANN, GB, vol. 30, no. 1, 1 January 2008 (2008-01-01), pages 84-90, XP022422788, ISSN: 1350-4533, DOI: 10.1016/J.MEDENGPHY.2006.12.001 * the whole document * ----- | 1-16 | |
| A | YUENG DELAHOZ ET AL: "Survey on Fall Detection and Fall Prevention Using Wearable and External Sensors", SENSORS, vol. 14, no. 10, 22 October 2014 (2014-10-22), pages 19806-19842, XP055371854, CH ISSN: 1424-8220, DOI: 10.3390/s141019806 * the whole document * ----- | 1-16 | |
| A | AMERICAN GERIATRICS SOCIETY ET AL: "Guideline for the Prevention of Falls in Older Persons", JOURNAL OF THE AMERICAN GERIATRICS SOCIETY., vol. 49, no. 5, 1 May 2001 (2001-05-01), pages 664-672, XP055368716, US ISSN: 0002-8614, DOI: 10.1046/j.1532-5415.2001.49115.x * the whole document * ----- | 1-16 | TECHNICAL FIELDS SEARCHED (IPC) |

The present search report has been drawn up for all claims

| Place of search | Date of completion of the search | Examiner |
|---|---|---|
| Munich | 7 June 2017 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 15 0270

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2017

| Patent document cited in search report | | Publication date | Patent family member(s) | | Publication date |
|---|---|---|---|---|---|
| US 2016220153 | A1 | 04-08-2016 | CN<br>EP<br>JP<br>US<br>WO | 105530865 A<br>3043709 A1<br>2016529081 A<br>2016220153 A1<br>2015036245 A1 | 27-04-2016<br>20-07-2016<br>23-09-2016<br>04-08-2016<br>19-03-2015 |
| GB 2352815 | A | 07-02-2001 | NONE | | |
| US 2011295621 | A1 | 01-12-2011 | NONE | | |
| US 2016147959 | A1 | 26-05-2016 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7612681 B2
- WO 2009156936 A2
- US 2013023798 A
- US 2013303860 A
- EP 2329470 B1
- WO 2015036245 A1
- US 7541934 B2
- US 20100124737 A1
- WO 2014136112 A1
- US 2015148113 A
- US 2015260514 A
- US 8082122 B2

**Non-patent literature cited in the description**

- **Y. J. GSCHWIND ; S. EICHBERG ; H. R. MARSTON ; A. EJUPI ; H. DE ROSARIO ; M. KROLL ; M. DROBICS ; J. ANNEGARN ; R. WIECHING ; S. R. LORD.** ICT-based system to predict and prevent falls (iStoppFalls): study protocol for an international multicenter randomized controlled trial. *BMC Geriatr.,* August 2014, vol. 14 (1), 91
- Adherence to and effectiveness of an individually tailored home-based exercise program for frail older adults, driven by mobility monitoring: design of a prospective cohort study. **HILDE AE GERAEDTS ; WIEBREN ZIJLSTRA ; WEI ZHANG ; SJOERD BULSTRA ; MARTIN STEVENS.** BMC Public Health. 2014, vol. 14, 570
- **A. WEISS ; M. BROZGOL ; M. DORFMAN ; T. HERMAN ; S. SHEMA ; N. GILADI ; J. M. HAUSDORFF.** Does the Evaluation of Gait Quality During Daily Life Provide Insight Into Fall Risk? A Novel Approach Using 3-Day Accelerometer Recordings. *Neurorehabilitation and Neural Repair,* 2013, vol. 27 (8), 742